# EUROPEAN PATENT APPLICATION

(11) **EP 4 659 595 A1**
(43) Date of publication of application: **10.12.2025**
(21) Application number: 24180532.4
(22) Date of filing: 06.06.2024
(51) Int. Cl.: A24B 13/00, A24B 15/16, A61K 31/465, A61K 47/58, A24B 15/30, A24B 15/42

(54) **NICOTINE-POLYMER COMPLEX COMPRISING MULTIPLE NICOTINE FORMS**

(71) Applicant: Nicoventures Trading Limited, London WC2R 3LA (GB)
(72) Inventor: Zawadzki, Michael A, North Carolina (US); Darrow, Brandon S., North Carolina (US)
(74) Representative: Newcombe, Christopher David

(57) **Abstract**

A method of preparing a nicotine-polymer complex is provided herein, along with the nicotine-polymer complex and a corresponding oral product containing such a complex are provided herein. The method can include contacting an exchange resin with a mixture of nicotine freebase and protonated nicotine or can include washing a nicotine resin to remove at least a portion of the nicotine therefrom and contacting the washed nicotine resin with a mixture of nicotine freebase and protonated nicotine.

## Description

### FIELD OF THE DISCLOSURE

The present disclosure relates to products intended for human use. The products are configured for oral use and deliver substances such as flavors and/or active ingredients during use. Such products may include tobacco or a product derived from tobacco, or may be tobacco-free alternatives.

### BACKGROUND

There are many categories of products intended for oral use and enjoyment. For example, oral tobacco products containing nicotine, which is known to have both stimulant and anxiolytic properties, have been available for many years. Conventional formats for so-called "smokeless" tobacco products include moist snuff, snus, and chewing tobacco, which are typically formed almost entirely of particulate, granular, or shredded tobacco, and which are either portioned by the user or presented to the user in individual portions, such as in single-use pouches or sachets. See for example, the types of smokeless tobacco formulations, ingredients, and processing methodologies set forth in US Pat. Nos. 6,668,839 to Williams; 6,834,654 to Williams; 6,953,040 to Atchley et al.; 7,032,601 to Atchley et al.; and 7,694,686 to Atchley et al.; 7,810,507 to Dube et al.; 7,819,124 to Strickland et al.; 7,861,728 to Holton, Jr. et al.; 7,901,512 to Quinter et al.; 8,627,828 to Strickland et al.; and 11,246,334 to Atchley, each of which is incorporated herein by reference.

In addition, traditional tobacco materials and non-tobacco materials have been combined with other ingredients to form product formats distinct from traditional smokeless products, with example formats including lozenges, pastilles, gels, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al., each of which is incorporated herein by reference.

There is continuing interest in the development of new types of oral products that deliver advantageous sensorial or biological activity. Such products typically contain flavorants and/or active ingredients such as nicotine, caffeine, a botanical, or cannabidiol. The format of such products can vary, and include pouched products containing a powdered or granular composition, lozenges, pastilles, liquids, gels, emulsions, meltable compositions, and the like. See, for example, the types of products described in US Patent App. Pub. Nos. 2022/0160675 to Gerardi et al.; 2022/0071984 to Poole et al.; 2021/0378948 to Gerardi et al.; 2021/0330590 to Hutchens et al.; 2021/0186081 to Gerardi et al.; 2021/0177754 to Keller et al; 2021/0177043 to Gerardi et al.; 2021/0177038 to Gerardi et al.; 2021/0169867 to Holton, Jr. et al.; 2021/0169792 to Holton, Jr. et al.; 2021/0169132 to Holton, Jr. et al.; 2021/0169121 to St. Charles, and 2021/0169122 to St. Charles, each of which is incorporated herein by reference.

### BRIEF SUMMARY

The present disclosure relates to nicotine-polymer complexes comprising a resin treated with two or more different forms of nicotine, e.g., free-base nicotine and protonated nicotine and to products incorporating such nicotine-polymer complexes. Although not intending to be limited by theory, it is believed that by treating a resin with two or more different forms of nicotine, the properties of a product into which such a nicotine-polymer complex is incorporated can be modulated, e.g., balancing the nicotine release profile particularly under certain pH and/or ionic strength conditions. In some embodiments, the inclusion of a nicotine-polymer complex as provided herein within an oral product can allow for more controlled nicotine release, faster nicotine release, and/or more complete nicotine release from the resin during use than a comparable product comprising a nicotine-polymer complex comprising only free-base nicotine. Some such products are pouched products including an outer water-permeable pouch defining a cavity containing a composition comprising a water-soluble component capable of being released through the water-permeable pouch. In addition to the nicotine-polymer complex, the composition within the cavity can further comprise various components, including, but not limited to, flavorants, sweeteners, fillers, and the like.

In some embodiments, the composition within the cavity of the pouch is a smokeless tobacco product or nicotine replacement therapy product. In some embodiments, the composition comprises substantially no nicotine or tobacco. In some embodiments, the composition within the cavity of the pouch is a particulate material adapted for steeping or brewing (i.e., configured for liquid extraction), such as a tea or coffee material. Accordingly, in some embodiments, the composition within the cavity of the pouch can comprise a particulate or fibrous plant material such as would be found in various teas or tea variants. In some embodiments, the composition within the cavity can comprise a flavor component such that flavor can be added to a liquid (e.g., water).

The disclosure includes, without limitation, the following embodiments.

Embodiment 1: A method of preparing a nicotine-polymer complex, comprising contacting an exchange resin with a mixture of nicotine freebase and protonated nicotine.

Embodiment 2: The method of Embodiment 1, wherein the exchange resin comprises a cationic exchange resin.

Embodiment 3: The method of Embodiment 1 or 2, wherein the exchange resin comprises polacrilex.

Embodiment 4: A method of preparing a nicotine-polymer complex, comprising: washing a nicotine-polymer complex to remove at least a portion of nicotine present thereon and form a washed nicotine-polymer complex; and contacting the washed nicotine-polymer complex with a mixture of nicotine freebase and protonated nicotine.

Embodiment 5: The method of Embodiment 4, wherein the nicotine-polymer complex comprises nicotine associated with a cationic exchange resin.

Embodiment 6: The method of Embodiment 4 or 5, wherein the nicotine-polymer complex comprises nicotine polacrilex.

Embodiment 7: The method of any of Embodiments 4 to 6, wherein the washing is done using water.

Embodiment 8: The method of any of Embodiments 4 to 7, wherein the washing is done using an acidic solution or an alkaline solution.

Embodiment 9: The method of any of Embodiments 1 to 8, wherein the protonated nicotine is in the form of a nicotine ion-pair.

Embodiment 10: The method of Embodiment 9, wherein the nicotine ion-pair is nicotine benzoate.

Embodiment 11: The method of any of Embodiments 1 to 8, wherein the protonated nicotine is in the form of a nicotine ion-pair complex.

Embodiment 12: The method of Embodiment 11, wherein the nicotine ion-pair complex is nicotine 3.2 molar excess benzoate.

Embodiment 13: The method of any of Embodiments 1 to 8, wherein the protonated nicotine is in the form of a nicotine salt.

Embodiment 14: The method of any of Embodiments 1 to 13, wherein the nicotine-polymer complex has a weight ratio of freebase nicotine to resin of 0 to 2.

Embodiment 15: The method of any of Embodiments 1 to 14, wherein the nicotine-polymer complex has a weight ratio of freebase nicotine to resin of 0 to 0.05.

Embodiment 16: The method of any of Embodiments 1 to 15, further comprising preparing the mixture of nicotine freebase and protonated nicotine.

Embodiment 17: The method of Embodiment 16, wherein the preparing comprises mixing the nicotine freebase and the protonated nicotine.

Embodiment 18: The method of Embodiment 16, wherein the preparing comprises mixing the nicotine freebase and an agent capable of reacting with nicotine freebase to give the protonated nicotine.

Embodiment 19: The method of any of Embodiments 1 to 18, wherein the contacting step further comprises heating and/or agitation.

Embodiment 20: The method of any of Embodiments 1 to 19, wherein the mixture further comprises one or more processing aids selected from the group consisting of salts, humectants, buffers, and combinations thereof.

Embodiment 21: The method of any of Embodiments 1 to 20, wherein the nicotine-polymer complex exhibits extended nicotine release when in a consumer's oral cavity.

Embodiment 22: A nicotine-polymer complex, prepared by the method of any of Embodiments 1 to 21.

Embodiment 23: A method of preparing a composition adapted for oral use within a pouched product, comprising: mixing a nicotine-polymer complex prepared according to the method of any of Embodiments 1 to 21 with one or more fillers and optional additional components to give the composition; and incorporating the composition within a cavity within an outer water-permeable pouch.

Embodiment 24: A pouched product comprising a composition adapted for oral use prepared according to the method of Embodiment 23 contained within a cavity included therein.

Embodiment 25: A pouched product comprising a nicotine polymer complex contained in a composition adapted for oral use in a cavity included therein, the pouched product exhibiting extended nicotine release (i.e., over a period of at least 30 minutes or at least 60 minutes).

Embodiment 26: A pouched product comprising a nicotine-polymer complex contained in a composition adapted for oral use in a cavity included therein, the pouched product exhibiting extended release as compared to a corresponding pouched product comprising a nicotine resin comprising only free base nicotine in place of the nicotine-polymer complex.

Embodiment 27: The pouched product of any of Embodiments 24-26, wherein 80% or more of the nicotine in the nicotine polymer complex is released from the oral product after 90 minutes of use.

Embodiment 28: The pouched product of any of Embodiments 24 to 27, having a pH of 8 or greater.

Embodiment 29: The pouched product of any of Embodiments 24 to 27, having a pH of 6.5 to 8.

Embodiment 30: The pouched product of any of Embodiments 24 to 27, having a pH of 6.5 or below.

Embodiment 31: The pouched product of any of Embodiments 24 to 30, wherein the nicotine-polymer complex is a first nicotine component and the composition further comprises a second nicotine component selected from the group consisting of nicotine freebase, protonated nicotine, and a nicotine salt.

Embodiment 32: The pouched product of any of Embodiments 24 to 31, wherein a total water content of the pouched product is 5% or greater, 10% or greater, 15% or greater, 25% or greater, or 30% or greater by weight, based on the total weight of the pouched product.

Embodiment 33: The pouched product of any of Embodiments 24 to 32, wherein a total water content of the pouched product is 80% or less, 70% or less, 60% or less, 50% or less 48% or less, 40% or less, 30% or less, 25% or less, 20% or less, 15% or less, or 10% or less, based on the total weight of the pouched product.

Embodiment 34: The pouched product of any of Embodiments 24 to 33, wherein the composition comprises one or more components selected from the group consisting of one or more fillers, binders, colorants, disintegration aids, antioxidants, humectants, and preservatives.

Embodiment 35: A nicotine-polymer complex comprising an exchange resin and a mixture of nicotine freebase and protonated nicotine.

Embodiment 36: A composition adapted for oral use comprising the nicotine-polymer complex of Embodiment 35, one or more fillers, and optional additional components.

Embodiment 37: A pouched product comprising the composition of Embodiment 36 within a cavity of an outer water-permeable pouch.

In some embodiments, the methods and pouched products of the Embodiments provided above relate to nicotine-polymer complexes exhibiting extended nicotine release when in a consumer's oral cavity.

These and other features, aspects, and advantages of the disclosure will be apparent from a reading of the following detailed description together with the accompanying drawings, which are briefly described below. The disclosure includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosure, in any of its various aspects and embodiments, should be viewed as intended to be combinable unless the context clearly dictates otherwise.

### BRIEF DESCRIPTION OF THE DRAWINGS

Having thus described aspects of the disclosure in the foregoing general terms, reference will now be made to the accompanying drawing, which is not necessarily drawn to scale. The drawing is an example only, and should not be construed as limiting the disclosure.
FIG. 1 is a depiction of an example method for preparing a nicotine-polymer complex according to some embodiments of the present disclosure;
FIGS. 2A and 2B are depictions of example methods for preparing a mixture that can be used for preparing a nicotine-polymer complex according to some embodiments of the present disclosure;
FIG. 3 is a depiction of an example method for preparing a nicotine-polymer complex according to some embodiments of the present disclosure;
FIG. 4 is a front perspective view illustrating a non-limiting pouched product according to an embodiment of the present disclosure; and
FIG. 5 is a graph of nicotine release over time for certain pouched products including a nicotine-polymer complex according to some embodiments of the present disclosure.

### DETAILED DESCRIPTION

The present disclosure now will be described more fully hereinafter. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. As used in this specification and the claims, the singular forms "a," "an," and "the" include plural referents unless the context clearly dictates otherwise.

Nicotine-polymer complexes are a common nicotine source in a range of oral products. Generally, a nicotine-polymer complex comprises a polymer comprising acidic sites, wherein at least a portion of the acidic sites are ionically bonded to nicotine counterions. The polymer of a nicotine-polymer complex can be any polymer (including homopolymers or all types of copolymers) with acidic functionalities, e.g., a polymeric cationic exchange resin. In some embodiments, the polymer comprises acidic sites that can be classified as strongly acidic, weakly acidic, or of intermediate acidity (depending, e.g., on the strength of the acid from which they are derived). In some embodiments, the polymer comprises weakly acidic sites and can be referred to as a weakly acidic cationic exchange resin (e.g., including, but not limited to, DuPont^{™} Amberlite^{™} IRP64). In some embodiments, the polymer comprises strongly acidic sites and can be referred to as a strongly acidic cationic exchange resin (e.g., including, but not limited to, DuPont^{™} Amberlite^{™} IRP69). Non-limiting examples of acidic sites include, *e.g.,* carboxylic acids, sulfonic acids, phosphonous acids, phophonic acids, phosphoric acids, iminodiacetic acids, and phenolic groups *(e.g.,* as disclosed in Adams et al., J. Soc. Chem. Ind. 54, IT (1935), which is incorporated herein by reference). Suitable polymers include, but are not limited to, addition polymers of styrene and divinylbenzene, divinylbenzene and methacrylic acid, divinylbenzene and acrylic acid, phenolic resins, or cellulose, dextran or pectin cross-linked with, e.g., epichlorohydrine. In some embodiments, the polymer comprises cross-linked moieties. Various such exchange resins are known in the art, including, but not limited to, DuPont^{™} Amberlite^{™} IRP64, DuPont^{™} Amberlite^{™} IRP69, Purolite^{™} C115HMR, or Doshion^{™} P551. *See, for example,* US Pat. No. 3,901,248 to Lichtneckert et al.*,* which is incorporated herein by reference. In some embodiments, exchange resins in salt form can be used, *e.g.,* including, but not limited to, polacrilin potassium, which is available as DuPont^{™} Amberlite^{™} IRP88, Kyron^{®} 314, and Purolite^{®} C115 KMR. Polacrilin potassium is the potassium salt of a low-crosslinked and weakly acidic cationic exchange resin prepared from methacrylic acid and divinylbenzene (which is then neutralized with potassium hydroxide).

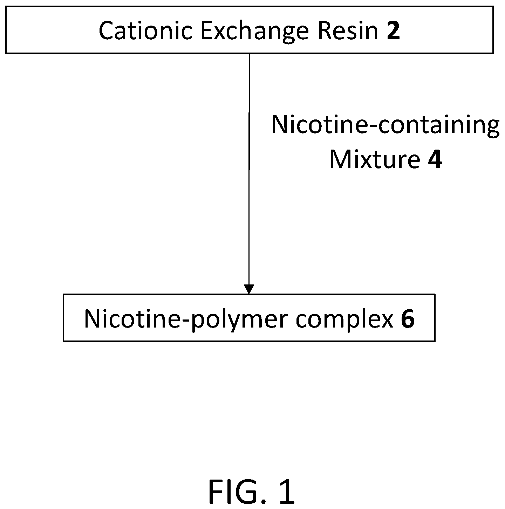

According to some embodiments of the present disclosure, a nicotine-polymer complex **6** is prepared by treating a cationic exchange resin **2** *(e.g.,* the types or resins outlined herein above) with a nicotine-containing mixture **4,** as schematically depicted in FIG. 1. The mixture **4** comprises at least one form of nicotine, as will be described further herein below and can further comprise various additional components (which may vary depending, *e.g.,* on the form(s) of nicotine contained within the mixture and the resin **2** being treated). In some embodiments, mixture **4** is in liquid form *(e.g.,* in the form of a solution, suspension, dispersion, or emulsion), such that mixture **4** further comprises a "solvent," which can be any liquid in which the components of the mixture are substantially soluble or dispersible at the temperature of the treating step *(e.g.,* room temperature). Examples of suitable solvents for mixture **4** include, but are not limited to, water, ether, alcohols (*e.g.,* methanol, ethanol, isopropanol, and the like), and mixtures thereof. In some embodiments, the solvent comprises water. In some embodiments, no solvent is employed and the mixture is provided neat or in solid form. The treating is generally conducted at room temperature (around 20°C), although in some embodiments, it may be beneficial to heat the mixture to promote complexation of the nicotine to the resin. As such, the temperature at which the treatment is conducted is not particularly limited and may, in some embodiments, be dependent upon the properties of the resin and/or the nicotine-containing mixture employed. In some embodiments, the temperature of this step can be in the range of 20°C to 60°C, *e.g.,* 30°C to 50°C and can optionally be varied during the step, *e.g.,* starting with an elevated temperature and reducing the temperature. Similarly, the pressure at which the treatment is conducted is not particularly limited and the treatment can, in some embodiments, be conducted under reduced pressure. Treatment can optionally further comprise agitation or stirring to promote complexation of the nicotine to the resin.

Mixture **4** generally comprises two or more forms of nicotine, *e.g.,* nicotine freebase and protonated nicotine. A first nicotine form included within some embodiments of mixture **4** is nicotine free base (also referred to as "freebase" or "free-base" nicotine), which is the conjugate base (deprotonated) form of nicotine. *See,* for example, the discussion of nicotine in freebase form in US Pat. Pub. No. 2004/0191322 to Hansson, which is incorporated herein by reference. A second nicotine form included within some embodiments of mixture **4** is "protonated nicotine." By "protonated nicotine" is meant any cationic form of nicotine, *e.g.,* including, but not limited to, a nicotine cation, a nicotine ion pair (such as nicotine benzoate), a nicotine ion pair complex (such as nicotine w/ molar excess benzoate), a nicotine salt, and the like.

Protonated nicotine in the form of a nicotine ion pair is nicotine associated, by ion pairing, with one or more organic acids or alkali or alkaline earth metal salt thereof . In some embodiments, the association between the nicotine and at least a portion of the organic acid or an alkali metal salt or alkaline earth metal salt thereof, is in the form of an ion pair between the basic amine and a conjugate base of the organic acid. Ion pairing describes the partial association of oppositely charged ions in relatively concentrated solutions to form distinct chemical species called ion pairs. The strength of the association (*i.e.,* the ion pairing) depends on the electrostatic force of attraction between the positive and negative ions (*i.e.,* protonated basic amine and the conjugate base of the organic acid). By "conjugate base" is meant the base resulting from deprotonation of the corresponding acid (*e.g.,* benzoate is the conjugate base of benzoic acid). On average, a certain population of these ion pairs exists at any given time, although the formation and dissociation of ion pairs is continuous.

In some embodiments as disclosed herein, mixture **4** comprises nicotine and the conjugate base of the organic acid at least partially in the form of an ion pair. One of skill in the art will recognize that the extent of ion pairing in the disclosed mixture **4** may vary based on, for example, pH, the nature of the organic acid, the concentration of the nicotine, the concentration of the organic acid or conjugate base of the organic acid present in the mixture, the ionic strength of the mixture, the other components of the mixture, and the like. One of skill in the art will also recognize that ion pairing is an equilibrium process influenced by the foregoing variables. Accordingly, quantification of the extent of ion pairing is difficult or impossible by calculation or direct observation. However, the presence of ion pairing may be demonstrated through surrogate measures such as partitioning between octanol and water or membrane permeation of aqueous solutions of *e.g.,* nicotine plus organic acids and/or their conjugate bases. Particularly, an octanol-water partitioning favoring distribution of a basic amine-organic acid ion pair into octanol is predictive of good absorption of the basic amine present in the composition through the oral mucosa. Advantageously, for purposes of the present disclosure, the properties of the mixture **4** (*e.g.,* pH) are tailored so as to maintain the desired amount of nicotine in the form of an ion-pair with one or more organic acids.

The organic acid associated with the referenced nicotine ion pair is an organic (*i.e.,* carbon-based) compound that is characterized by acidic properties. Typically, organic acids are relatively weak acids (*i.e.,* they do not dissociate completely in the presence of water), such as carboxylic acids (-CO₂H) or sulfonic acids (-SO₂OH). Suitable organic acids will typically have a range of lipophilicities (*i.e.,* a polarity giving an appropriate balance of water and organic solubility). Lipophilicity is conveniently measured in terms of logP, the partition coefficient of a molecule between a lipophilic phase and an aqueous phase (usually water). Typically, the lipophilic phase is octanol, although other lipophilic solvents may also be used. For avoidance of doubt, reference in the present disclosure to logP means the partition coefficient between octanol and water. Similarly, reference to a logD value herein means a logD obtained by partition between octanol and water (buffered to be at a specific pH value). %

Typically, lipophilicities of suitable organic acids, as indicated by logP, will vary between 1 and 12 (more soluble in octanol than in water). In some embodiments, the organic acid has a logP value from 1 to 12, e.g., from 1.0. 1.5, 2.0, 2.5, 3.0, 3.5, 4.0, 4.5, 5.0, 5.5, 6.0, 6.5, 7.0, 7.5, or 8.0, to 8.5, 9.0, 9.5, 10.0, 10.5, 11.0, 11.5, or 12.0. Without wishing to be bound by theory, it is believed that moderately lipophilic organic acids (e.g., logP of from 1.4 to 4.5) produce ion pairs with nicotine which are of a polarity providing good octanol-water partitioning of the ion pair, and hence partitioning of nicotine, into octanol versus water. As discussed above, such partitioning into octanol is predictive of favorable oral availability. In some embodiments, the organic acid has a logP value from 3.0 to 8.0, 10.0, or 12.0. In some embodiments, the presence of certain solvents or solubilizing agents in mixture **4** *(e.g.,* inclusion in the composition of glycerin or propylene glycol) may be beneficial in solubilizing organic acids and the corresponding salts or ion pairs thereof with the nicotine for highly lipophilic organic acids (*e.g.,* higher than 4.5).

In some embodiments, a hydrophilic acid is chosen so as to increase water solubility and/or decrease lipophilicity of the salt. Lipophilicity of a substituted 3-(1-methylpyrrolidin-2-yl)pyridine salt can also be expressed as logD, which is the logarithm of the distribution coefficient, a measure of the pH-dependent differential solubility between an octanol phase and an aqueous phase of all species (ionized and un-ionized) in an octanol/aqueous system, represented by the formula:

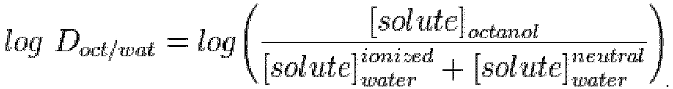

LogD is a commonly used descriptor for the lipophilicity of ionizable compounds. LogD values can be calculated using commercial software or may be determined experimentally in a similar manner to logP but instead of using water, the aqueous phase is adjusted to a specific pH using a buffer. LogD is pH dependent and therefore requires that the pH at which the logD was measured be specified.

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms (C₁-C₂₀). In some embodiments, the organic acid is an alkyl, cycloalkyl, heterocycloalkyl, aryl, or heteroaryl carboxylic or sulfonic acid.

As used herein, "alkyl" refers to any straight chain or branched chain hydrocarbon. The alkyl group may be saturated (i.e., having all *sp³* carbon atoms), or may be unsaturated (i.e., having at least one site of unsaturation). As used herein, the term "unsaturated" refers to the presence of a carbon-carbon, *sp²* double bond in one or more positions within the alkyl group. Unsaturated alkyl groups may be mono- or polyunsaturated. Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl. Representative unsaturated alkyl groups include, but are not limited to, ethylene or vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. An alkyl group can be unsubstituted or substituted. "Cycloalkyl" as used herein refers to a carbocyclic group, which may be mono- or bicyclic. Cycloalkyl groups include rings having 3 to 7 carbon atoms as a monocycle or 7 to 12 carbon atoms as a bicycle. Examples of monocyclic cycloalkyl groups include cyclopropyl, cyclobutyl, cyclopentyl, cyclohexyl, cycloheptyl, and cyclooctyl. A cycloalkyl group can be unsubstituted or substituted, and may include one or more sites of unsaturation (e.g., cyclopentenyl or cyclohexenyl).

The term "aryl" as used herein refers to a carbocyclic aromatic group. Examples of aryl groups include, but are not limited to, phenyl and naphthyl. An aryl group can be unsubstituted or substituted. "Heteroaryl" and "heterocycloalkyl" as used herein refer to an aromatic or nonaromatic ring system, respectively, in which one or more ring atoms is a heteroatom, e.g. nitrogen, oxygen, and sulfur. The heteroaryl or heterocycloalkyl group comprises up to 20 carbon atoms and from 1 to 3 heteroatoms selected from N, O, and S. A heteroaryl or heterocycloalkyl may be a monocycle having 3 to 7 ring members (for example, 2 to 6 carbon atoms and 1 to 3 heteroatoms selected from N, O, and S) or a bicycle having 7 to 10 ring members (for example, 4 to 9 carbon atoms and 1 to 3 heteroatoms selected from N, O, and S), for example: a bicyclo[4,5], [5,5], [5,6], or [6,6] system. Examples of heteroaryl groups include by way of example and not limitation, pyridyl, thiazolyl, tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, benzotriazolyl, benzisoxazolyl, and isatinoyl. Examples of heterocycloalkyls include by way of example and not limitation, dihydroypyridyl, tetrahydropyridyl (piperidyl), tetrahydrothiophenyl, piperidinyl, 4-piperidonyl, pyrrolidinyl, 2-pyrrolidonyl, tetrahydrofuranyl, tetrahydropyranyl, bis-tetrahydropyranyl, tetrahydroquinolinyl, tetrahydroisoquinolinyl, decahydroquinolinyl, octahydroisoquinolinyl, piperazinyl, quinuclidinyl, and morpholinyl. Heteroaryl and heterocycloalkyl groups can be unsubstituted or substituted.

"Substituted" as used herein and as applied to any of the above alkyl, aryl, cycloalkyl, heteroaryl, heterocyclyl, means that one or more hydrogen atoms are each independently replaced with a substituent. Typical substituents include, but are not limited to, -Cl, Br, F, alkyl, -OH, -OCH₃, NH₂, -NHCH₃, -N(CH₃)₂, -CN, -NC(=O)CH₃, -C(=O)-, -C(=O)NH₂, and -C(=O)N(CH₃)₂. Wherever a group is described as "optionally substituted," that group can be substituted with one or more of the above substituents, independently selected for each occasion. In some embodiments, the substituent may be one or more methyl groups or one or more hydroxyl groups.

In some embodiments, the organic acid is an alkyl carboxylic acid. Non-limiting examples of alkyl carboxylic acids include formic acid, acetic acid, propionic acid, butyric acid, valeric acid, caproic acid, heptanoic acid, octanoic acid, nonanoic acid, decanoic acid, undecanoic acid, dodecanoic acid, stearic acid, oleic acid, linoleic acid, linolenic acid, and the like. In some embodiments, the organic acid is an alkyl sulfonic acid. Non-limiting examples of alkyl sulfonic acids include propanesulfonic acid, heptanesulfonic acid, and octanesulfonic acid. In some embodiments, the alkyl carboxylic or sulfonic acid is substituted with one or more hydroxyl groups. Non-limiting examples include glycolic acid, 4-hydroxybutyric acid, and lactic acid. In some embodiments, an organic acid may include more than one carboxylic acid group or more than one sulfonic acid group *(e.g.,* two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like. In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid,and *p*-toluenesulfonic acid.

Further non-limiting examples of organic acids which may be useful in some embodiments include 2,2-dichloroacetic acid, 2-hydroxyethanesulfonic acid, 2-oxoglutaric acid, 4-acetamidobenzoic acid, 4-aminosalicylic acid, adipic acid, ascorbic acid (L), aspartic acid (L), alpha-methylbutyric acid, camphoric acid (+), camphor-10-sulfonic acid (+), cinnamic acid, cyclamic acid, dodecylsulfuric acid, ethane-1,2-disulfonic acid, ethanesulfonic acid, furoic acid, galactaric acid, gentisic acid, glucoheptonic acid, gluconic acid, glucuronic acid, glutamic acid, glycerophosphoric acid, glycolic acid, hippuric acid, isobutyric acid, isovaleric acid, lactobionic acid, lauric acid, levulinic acid, malic acid, malonic acid, mandelic acid, methanesulfonic acid, naphthalene-1,5-disulfonic acid, naphthalene-2-sulfonic acid, oleic acid, palmitic acid, pamoic acid, phenylacetic acid, pyroglutamic acid, pyruvic acid, sebacic acid, stearic acid, and undecylenic acid.

Examples of suitable acids include, but are not limited to, the list of organic acids in Table 1.

**Table 1. Non-limiting examples of suitable organic acids**

| **Acid Name** | **logP** |
|---|---|
| benzoic acid | 1.9 |
| phenylacetic | 1.4 |
| p-toluic acid | 2.3 |
| ethyl benzoic acid | 2.9 |
| isopropyl benzoic acid | 3.5 |
| 4-phenylbutyric | 2.4 |
| 2-napthoxyacetic acid | 2.5 |
| napthylacetic acid | 2.7 |
| heptanoic acid | 2.5 |
| octanoic acid | 3.05 |
| nonanoic acid | 3.5 |
| decanoic acid | 4.09 |
| 9-deceneoic acid | 3.3 |
| 2-deceneoic acid | 3.8 |
| 10-undecenoic acid | 3.9 |
| dodecandioic acid | 3.2 |
| dodecanoic acid | 4.6 |
| myristic acid | 5.3 |
| palmitic acid | 6.4 |
| stearic acid | 7.6 |
| cyclohexanebutanoic acid | 3.4 |
| 1-heptanesulfonic acid | 2.0 |
| 1-octanesulfonic acid | 2.5 |
| 1-nonanesulfonic acid | 3.1 |
| monooctyl succinate | 2.8 |
| tocopherol succinate | 10.2 |
| monomenthyl succinate | 3 |
| monomenthyl glutarate | 3.4 |
| norbixin ((2E,4E,6E,8E,10E, 12E, 14E, 16E, 18E)-4,8,13, 17-tetramethylicosa-2,4,6,8,10,12,14,16,18-nonaenedioic acid) | 7.2 |
| bixin ((2E,4E,6E,8E,10E,12E,14E,16Z,18E)-20-methoxy-4,8,13,17-tetramethyl-20-oxoicosa-2,4,6,8,10,12,14,16,18-nonaenoic acid) | 7.5 |
| Dibenzoyl-tartaric acid | 2.6 |

In some embodiments, the organic acid is a mono ester of a di- or poly-acid, such as mono-octyl succinate, mono-octyl fumarate, or the like. For example, in some embodiments, the organic acid is a mono ester of a dicarboxylic acid or a poly-carboxylic acid. In some embodiments, the dicarboxylic acid is malonic acid, succinic acid, glutaric acid, adipic acid, fumaric acid, maleic acid, or a combination thereof. In some embodiments, the dicarboxylic acid is succinic acid, glutaric acid, fumaric acid, maleic acid, or a combination thereof. In some embodiments, the dicarboxylic acid is succinic acid, glutaric acid, or a combination thereof.

In some embodiments, the alcohol forming the mono ester of the dicarboxylic acid is a lipophilic alcohol. Examples of suitable lipophilic alcohols include, but are not limited to, octanol, menthol, and tocopherol. In some embodiments, the organic acid is an octyl mono ester of a dicarboxylic acid, such as monooctyl succinate, monooctyl fumarate, or the like. In some embodiments, the organic acid is a monomenthyl ester of a dicarboxylic acid. Certain menthyl esters may be desirable in oral compositions as described herein by virtue of the cooling sensation they may provide upon use of the product comprising the composition. In some embodiments, the organic acid is monomenthyl succinate, monomenthyl fumarate, monomenthyl glutarate, or a combination thereof. In some embodiments, the organic acid is a monotocopheryl ester of a dicarboxylic acid. Certain tocopheryl esters may be desirable in oral compositions as described herein by virtue of the antioxidant effects they may provide. In some embodiments, the organic acid is tocopheryl succinate, tocopheryl fumarate, tocopheryl glutarate, or a combination thereof.

In some embodiments, the organic acid is a carotenoid derivative having one or more carboxylic acids. Carotenoids are tetraterpenes, meaning that they are produced from 8 isoprene molecules and contain 40 carbon atoms. Accordingly, they are usually lipophilic due to the presence of long unsaturated aliphatic chains, and are generally yellow, orange, or red in color. Certain carotenoid derivatives can be advantageous in oral compositions by virtue of providing both ion pairing and serving as a colorant in the composition. In some embodiments, the organic acid is 2*E*,4*E*,6*E*,8*E*,10*E*,12*E*,14*E*,16*Z*,18*E*)-20-methoxy-4,8,13,17-tetramethyl-20-oxoicosa-2,4,6,8,10,12,14,16,18-nonaenoic acid (bixin) or an isomer thereof. Bixin is an apocarotenoid found in annatto seeds from the achiote tree (*Bixa orellana*), and is the naturally occurring pigment providing the reddish orange color to annatto. Bixin is soluble in fats and alcohols but insoluble in water, and is chemically unstable when isolated, converting via isomerization into the double bond isomer, *trans*-bixin (β-bixin), having the structure:

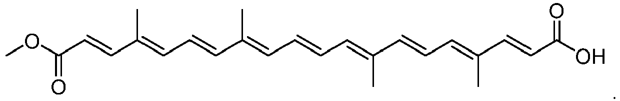

In some embodiments, the organic acid is (2*E*,4*E*,6*E*,*8*E,10*E*,12*E*,14*E*,16*E*,18*E*)-4,8,13,17-tetramethylicosa-2,4,6,8,10,12,14,16,18-nonaenedioic acid (norbixin), a water soluble hydrolysis product of bixin having the structure:

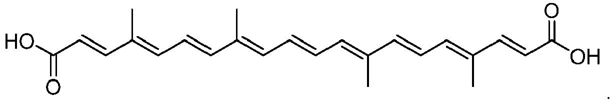

The selection of organic acid may further depend on additional properties in addition to or without consideration to the logP value. For example, an organic acid should be one recognized as safe for human consumption, and which has acceptable flavor, odor, volatility, stability, and the like. Determination of appropriate organic acids is within the purview of one of skill in the art. In some embodiments, the organic acid is benzoic acid, a toluic acid, benzenesulfonic acid, toluenesulfonic acid, hexanoic acid, heptanoic acid, decanoic acid, or octanoic acid. In some embodiments, the organic acid is benzoic acid, octanoic acid, or decanoic acid. In some embodiments, the organic acid is octanoic acid. In some embodiments, the organic acid is benzoic acid.

In some embodiments, more than one organic acid may be present. For example, the organic acid may comprise two, or three, or four, or more organic acids. Accordingly, reference herein to "an organic acid" contemplates mixtures of two or more organic acids. The relative amounts of the multiple organic acids may vary. For example, nicotine ion pairs as described herein may comprise equal amounts of two, or three, or more organic acids, or may comprise different relative amounts. In this manner, it is possible to include certain organic acids (e.g., citric acid or myristic acid) which have a logP value outside the desired range, when combined with other organic acids to provide the desired average logP range for the combination. In some embodiments, it may be desirable to include organic acids which have logP values outside the desired range for purposes such as, but not limited to, providing desirable organoleptic properties, stability, as flavor components, and the like. Further, certain lipophilic organic acids have undesirable flavor and or aroma characteristics which would preclude their presence as the sole organic acid *(e.g.,* in equimolar or greater quantities relative to nicotine). Without wishing to be bound by theory, it is believed that a combination of different organic acids may provide desirable ion pairing while the concentration of any single organic acid in the mixture **4** (and, ultimately, in the oral composition/product into which the nicotine-polymer complex provided herein is incorporated) remains below the threshold which would be found objectionable from a sensory perspective. For example, in some embodiments, the organic acid may comprise from 1 to 5 or more molar equivalents of benzoic acid relative to the basic amine-containing active ingredient *(e.g.,* nicotine), combined with *e.g.,* 0.2 molar equivalents of octanoic acid or a salt thereof, and 0.2 molar equivalents of decanoic acid or a salt thereof.

In some embodiments, the organic acid is a combination of any two organic acids selected from the group consisting of benzoic acid, a toluic acid, benzenesulfonic acid, toluenesulfonic acid, hexanoic acid, heptanoic acid, decanoic acid, and octanoic acid. In some embodiments, the organic acid is a combination of benzoic acid, octanoic acid, and decanoic acid, or benzoic and octanoic acid. In some embodiments, the composition comprises citric acid in addition to one or more of benzoic acid, a toluic acid, benzenesulfonic acid, toluenesulfonic acid, hexanoic acid, heptanoic acid, decanoic acid, and octanoic acid.

The amount of organic acid or salt thereof relative to the nicotine may vary. Generally, as the concentration of the organic acid (or the conjugate base thereof) increases, the percent of nicotine that is ion paired with the organic acid increases. This typically increases the partitioning of the basic amine-containing active ingredient (*e.g.,* nicotine), in the form of an ion pair, into octanol versus water as measured by the logP (the log₁₀ of the partitioning coefficient). In some embodiments, the mixture **4** comprises from 0.05, 0.1, 1, 1.5, 2, or 5, to 10, 15, or 20 molar equivalents of the organic acid, the salt thereof, or the combination thereof, relative to the nicotine, calculated as the free base.

In some embodiments, the composition comprises from 1 to 10, or from 2 to 5 molar equivalents of the organic acid, the salt thereof, or the combination thereof, to nicotine, on a free-base nicotine basis (referred to as a "nicotine ion-pair complex"). In some embodiments, the organic acid, the salt thereof, or the combination thereof, is present in a molar ratio with nicotine from 1, 2, 3, 4, or 5, to 6, 7, 8, 9, or 10. In some embodiments, a nicotine ion-pair complex comprising nicotine and a 3.2 molar excess acid, *e.g.,* benzoate, is used. In embodiments wherein more than one organic acid, salt thereof, or both, are present, it is to be understood that such molar ratios reflect the totality of the organic acids present.

Protonated nicotine in the form of a nicotine salt is a salt of nicotine with one or more organic acids. Salts of nicotine can be provided using the types of ingredients and techniques set forth in US Pat. No. 2,033,909 to Cox et al. and Perfetti, Beitrage Tabakforschung Int., 12: 43-54 (1983), which are incorporated herein by reference. Additionally, salts of nicotine are available from sources such as Pfaltz and Bauer, Inc. and K&K Laboratories, Division of ICN Biochemicals, Inc. Further salts of nicotine are described in U.S. Patent Nos. 9,738,622; 9,738,622; 9,896,429; 10,464,917; 10,508,096; 10,556,880; 10,865,192; and 11,136,305, all to RJ Reynolds, which are incorporated herein by reference in their entireties. References to "nicotine salt" as used herein are also intended to cover nicotine co-crystals and salt-co-crystal complexes, which may vary in the interaction between the nicotine and its coformer, *e.g.,* as described in the patents to RJ Reynolds referenced herein above. As such, "nicotine salt" as used herein encompasses forms of nicotine and at least one other coformer, wherein both nicotine and coformer are in ionic form (nicotine salt); forms of nicotine and at least one other coformer, wherein both nicotine and coformer are in neutral form (nicotine co-crystal); and hybrid bonding structures with both salt and co-crystal characteristics (nicotine salt-co-crystals), unless the context dictates otherwise. The nicotine salt is, in some embodiments, selected from the group consisting of a nicotine salt selected from the group consisting of nicotine hydrochloride, dihydrochloride, monotartrate, bitartrate, sulfate, salicylate, and nicotine zinc chloride. As referenced above with respect to nicotine ion-pairs and nicotine ion-pair complexes, nicotine salts can be prepared prior to inclusion within mixture **4** or can be formed in-situ, *i.e.,* within mixture **4.**

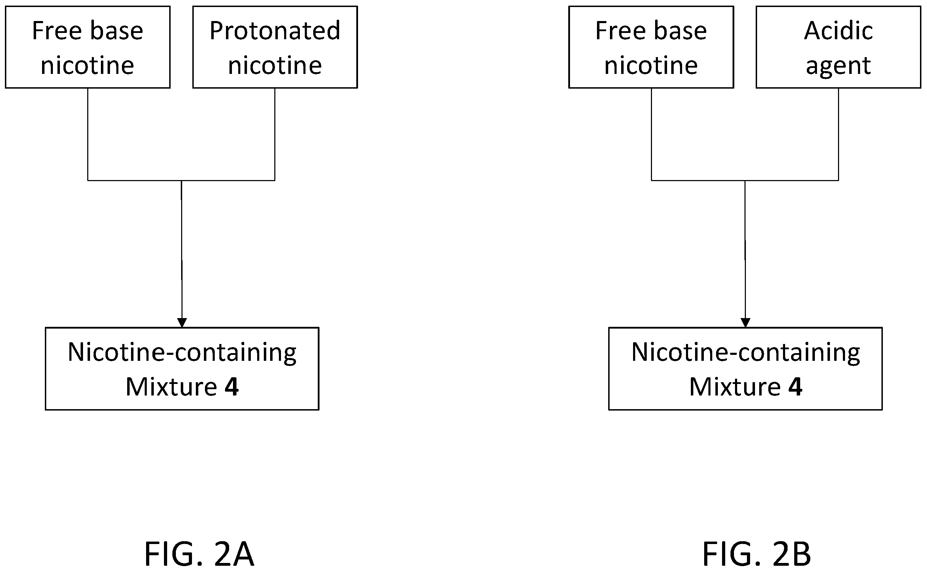

As shown in FIGS. 2A and 2B, mixture **4** can be prepared by combining the nicotine freebase and the protonated nicotine (FIG. 2A) or by combining nicotine freebase with an agent capable of forming protonated nicotine (*e.g.,* an organic acid) (FIG. 2B). In other words, the mixture **4** is prepared using both nicotine freebase and protonated nicotine or is prepared using freebase nicotine and an agent capable of forming protonated nicotine in situ, *i.e.,* within the mixture.

According to the method depicted in FIG. 2A, the mixture **4** is prepared with freebase nicotine and protonated nicotine. The protonated nicotine in some embodiments is prepared and included directly within mixture **4,** *e.g.,* based on known methods for the production of nicotine ion-pairs, nicotine ion-pair complexes, and nicotine salts.

According to the method depicted in FIG. 2B, the mixture **4** is prepared with a freebase nicotine and an acidic agent (*e.g.,* an organic acid and/or pH adjuster capable of forming protonated nicotine (*e.g.,* an acid such as hydrochloric acid, benzoic acid, or the like)). To form mixture **4** according to some embodiments, the acidic agent, *e.g.,* organic acid, can be added as the free acid or as a salt form of the acid. The freebase nicotine and the acidic agent can independently be employed in neat (*i.e.,* native solid or liquid) form or as a solution, dispersion, suspension, or emulsion. The free base nicotine and the acidic agent are combined with any optional additional components of mixture **4** in any order, such that protonated nicotine (*e.g.,* in the form of a nicotine ion pair, nicotine ion-pair complex, or nicotine salt) is formed in situ within mixture **4).** In some embodiments, the mixture **4** further comprises a solubility enhancer to increase the solubility of one or more of the organic acid or salt thereof. Suitable solubility enhancers include, but are not limited to, humectants as described herein such as glycerin or propylene glycol. In the method depicted in FIG. 2B, the conditions (*e.g.,* component concentrations within the mixture, mixture pH, mixture temperature) are advantageously controlled so as to promote formation of the desired amount of protonated nicotine.

The molar ratio of nicotine freebase and protonated nicotine in mixture **4** can vary. In some embodiments, mixture **4** comprises a higher concentration of freebase nicotine than protonated nicotine immediately prior to contact with the resin. In some embodiments, mixture **4** comprises a lower concentration of freebase nicotine than protonated nicotine immediately prior to contact with the resin. In some embodiments, mixture **4** comprises a roughly equivalent concentration (*e.g.,* 1:1 molar ratio) of freebase nicotine than protonated nicotine immediately prior to contact with the resin. After the resin is treated with the mixture **4,** it can be directly used (*e.g.,* incorporated within an oral product as provided herein below), stored, or further processed (*e.g.,* including, but not limited to, via washing, drying, and the like).

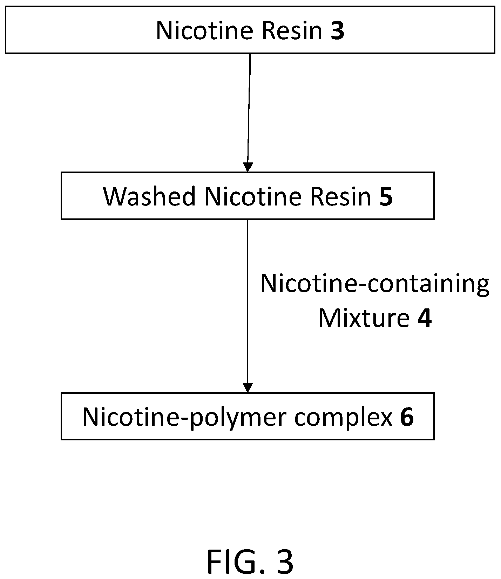

In a further embodiment of the present disclosure, a nicotine-polymer complex **6** is prepared by treating a nicotine resin **3** with a nicotine-containing mixture **4,** as schematically depicted in FIG. 3. "Nicotine resin" starting materials as provided herein are nicotine-polymer complexes, but referred to in various places throughout the present disclosure as "nicotine resins" to distinguish them from the "nicotine-polymer complexes" described herein. Certain nicotine resins are commercially available, *e.g.,* a nicotine-polyacrylic carbomer complex, such as Carbopol 974P or nicotine polacrilex, which comprises nicotine bound to a resin prepared from methacrylic acid and divinyl benzene (available in varying nicotine percentages, *e.g.,* 18% to 20% nicotine). Generally, nicotine resin **3** is first washed. The washing can be conducted using, *e.g.,* an acidic solution, an alkaline solution, or substantially neutral aqueous solution. Advantageously, the washing is conducted to decrease the nicotine concentration on the nicotine resin **3,** providing a washed nicotine resin **5** comprising less nicotine associated with the resin than in untreated nicotine resin **3.** The washing can be conducted in various ways and may optionally involve heating, agitation, and/or the like to promote dissociation of at least a portion of the nicotine from the resin. The washing can be conducted once or two times or more (using the same or different solutions). Following the washing step, the washed nicotine resin **5** can optionally be dried (e.g., under heat and/or vacuum). Following the washing (and optional drying) of the nicotine resin, the washed nicotine resin **5** is treated with a nicotine-containing mixture **4** as provided above, *i.e.,* comprising freebase nicotine and protonated nicotine. Again, the mixture **4** can be pre-prepared *(e.g.,* according to the method depicted in FIG. 2A) or prepared in situ (*e.g.,* according to the method depicted in FIG. 2B).

The methods disclosed above can, in some embodiments, provide a unique nicotine-polymer complex **6.** In some embodiments, the free-base nicotine to resin weight ratio of the nicotine-polymer complex **6** is 0 to 2, e.g., 0 to 1, 0 to 0.5, 0 to 0.25, 0 to 0.1, or 0 to 0.05. In some embodiments, the nicotine polymer complex **6** comprises a greater percentage of sites on the polymer having a nicotine cation associated therewith (*e.g.,* as compared to conventional nicotine-polymer complexes (e.g., nicotine resins such as those referenced above) and as compared to a comparable untreated nicotine resin or to a comparable resin treated under substantially similar conditions with nicotine freebase alone.

The nicotine-polymer complex provided according to the methods referenced herein can be used directly or can be further processed prior to inclusion in products, *e.g.,* oral products. In some embodiments, following the disclosed treatment, the nicotine-polymer complex **6** is further processed. For example, in some embodiments, the further processing comprises drying the nicotine-polymer complex **6** and/or separating it from liquid, *e.g.,* by filtration *(e.g.,* vacuum filtration) or the like. In some embodiments, further processing can comprise washing the nicotine-polymer complex **6** with a solvent (such as water) and/or additional mixture **4** (comprising further nicotine freebase and protonated nicotine). In some embodiments, multiple washing steps can be conducted. In some embodiments, further processing can comprise drying the nicotine polymer complex **6.** In some embodiments, the drying is done in an oven (*e.g.,* convection oven) at elevated temperature (*e.g.,* above 20°C, such as 20°C to 60°C). The time for which the material is dried can be that time to achieve the desired moisture content.

The amount of the nicotine-polymer complex included within a given oral product can vary. Certain, non-limiting example amounts of nicotine-polymer complex incorporated within a given composition (*e.g.,* for inclusion within a pouched product) can range, *e.g.,* from 0.5% by weight to 15% by weight, *e.g.,* 1% by weight to 10% by weight, *e.g.,* 2% to 5% by weight, based on the total weight of the composition/mixture to be included within a pouched product. It is to be understood that the amount of the nicotine-polymer complex to be included within a given oral product can depend, e.g., on the content of nicotine within the nicotine-polymer complex and the content of any other optional nicotine sources within the oral product.

In some embodiments, the nicotine-polymer complex disclosed herein is the sole source of nicotine in the compositions and products. In some embodiments, the compositions and products comprise at least two, different nicotine components (including a nicotine-polymer complex as described herein). It is noted that this disclosure is not intended to be limited thereto, and in some embodiments, such compositions and products can include more than two, different nicotine components (e.g., three, four, or more different nicotine components), including the nicotine-polymer complex as described herein. The amount of nicotine provided by the nicotine-polymer complex as described herein can vary from 5%, 10%, 20%, 30%, 40%, 50%, or 60% to 40%, 50%, 60%, 70%, 80%, 90%, 95%, or 100%, based on the total weight of nicotine provided from all nicotine components within a given composition. In some embodiments, the optional additional nicotine source(s) can be unbound nicotine (*e.g.,* nicotine freebase, ion-pair nicotine, ion-pair nicotine complex, or nicotine salt). In some embodiments, more than one nicotine-polymer complex is incorporated within the compositions and products described herein (wherein at least one such nicotine-polymer complex comprises a nicotine-polymer complex as described herein). In some embodiments, one or more unfunctionalized resins may be further incorporated within certain disclosed oral products.

The disclosure generally provides compositions and products configured for oral use. The term "configured for oral use" as used herein means that the product is provided in a form such that during use, saliva in the mouth of the user causes one or more of the components of the mixture (e.g., flavoring agents and/or nicotine) to pass into the mouth of the user. In some embodiments, the product is adapted to deliver components to a user through mucous membranes in the user's mouth, the user's digestive system, or both and, in some instances, said component is an active ingredient (including, but not limited to, for example, nicotine) that can be absorbed through the mucous membranes in the mouth or absorbed through the digestive tract when the product is used. Certain compositions or products of the present disclosure may be dissolvable. As used herein, the terms "dissolve," "dissolving," and "dissolvable" refer to compositions having aqueous-soluble components that interact with moisture in the oral cavity and enter into solution, thereby causing gradual consumption of the composition. According to one aspect, the dissolvable composition is capable of lasting in the user's mouth for a given period of time until it completely dissolves. Dissolution rates can vary over a wide range, from 1 minute or less to 60 minutes. For example, fast release compositions typically dissolve and/or release the desired component(s) (e.g., active ingredient, flavor, and the like) in 2 minutes or less, often 1 minute or less (e.g., 50 seconds or less, 40 seconds or less, 30 seconds or less, or 20 seconds or less). Dissolution can occur by any means, such as melting, mechanical disruption (e.g., chewing), enzymatic or other chemical degradation, or by disruption of the interaction between the components of the composition. In some embodiments, the products do not dissolve during the product's residence in the user's mouth.

In some embodiments, the disclosure provides products in the form of a mixture of one or more components (including a nicotine-polymer complex as described further herein), disposed within a moisture-permeable container (e.g., a water-permeable pouch). Pouched products generally comprise, in addition to the pouch-based exterior, a mixture within the pouch that typically comprises (in addition to the nicotine component(s) as described herein), one or more fillers, one or more flavorants, and various other optional ingredients. The composition of the material within the pouches provided herein is not particularly limited, and can comprise, in addition to the nicotine-polymer complex (alone or in combination with one or more different nicotine sources), any filling composition, including those included within conventional pouched produces. Such compositions are generally mixtures of two or more components and as such, the compositions are, in some cases, referenced herein below as "mixtures." Certain components that can advantageously be included in the mixtures within some embodiments of the pouches provided herein are outlined generally below; however, it is to be understood that the discussion is not intended to be limiting of the components that can be incorporated within the disclosed pouches.

Such mixtures in the water-permeable pouch format are typically used by placing a pouch containing the mixture in the mouth of a human subject/user. Generally, the pouch is placed somewhere in the oral cavity of the user, for example under the lips, in the same way as moist snuff products are generally used. The pouch typically is not chewed or swallowed. Exposure to saliva then causes some of the components of the mixture therein (e.g., flavoring agents and/or the nicotine) to pass through e.g., the water-permeable pouch and provide the user with flavor and satisfaction, and the user is not required to spit out any portion of the mixture. After 10 minutes to 60 minutes, e.g., 15 minutes to 45 minutes of use/enjoyment, substantial amounts of the mixture have been ingested by the human subject, and the pouch may be removed from the mouth of the consumer for disposal. In some embodiments, pouch materials for products described herein may be designed and manufactured such that under conditions of normal use, a significant amount of the contents of the formulation within the pouch permeate through the pouch material prior to the time that the pouch undergoes loss of its physical integrity.

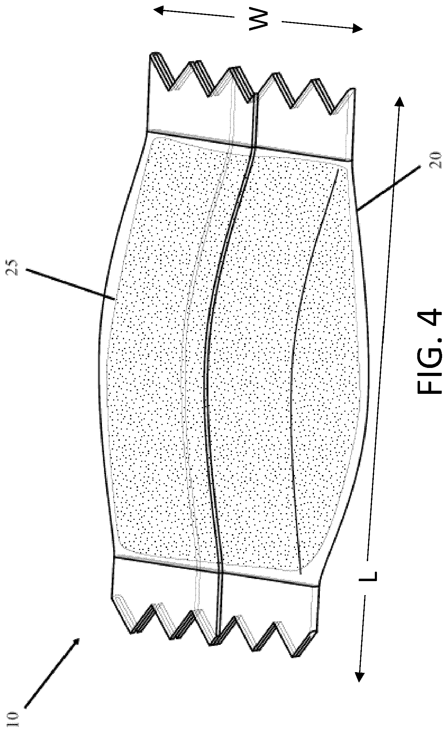

For example, as illustrated in FIG. 4, an example pouched product **10** can comprise an outer water-permeable container **20** in the form of a pouch which contains a particulate mixture **25** adapted for oral use (wherein particulate mixture **25** comprises a nicotine-polymer complex as described herein). The orientation, size, and type of outer water-permeable pouch and the type and nature of the composition adapted for oral use that are illustrated herein are not construed as limiting thereof. Certain examples of components incorporated within particulate mixture **25** are described herein below, followed by disclosure relating to suitable outer, water-permeable containers **20** to form pouched products **10.**

### Nicotine component

As referenced herein above, compositions and products are provided herein which contain at least one nicotine component in the form of a nicotine-polymer complex as described herein. In some embodiments, the nicotine-polymer complex is provided in particulate form with a given average particle size. In some embodiments, the nicotine-polymer complex within the disclosed compositions and products has an average particle size of at least 1 micrometer, at least 5 micrometers, at least 10 micrometers, at least 25 micrometers, at least 50 micrometers, at least 100 micrometers, at least 150 micrometers, at least 200 micrometers, at least 250 micrometers, at least 300 micrometers, at least 350 micrometers, at least 400 micrometers, or at least 450 micrometers. In some embodiments, the nicotine-polymer complex within the disclosed compositions and products has an average particle size of less than 800 micrometers, less than 750 micrometers, less than 700 micrometers, less than 650 micrometers, less than 600 micrometers, less than 550 micrometers, less than 500 micrometers, less than 450 micrometers, less than 400 micrometers, less than 350 micrometers, less than 300 micrometers, less than 250 micrometers, less than 200 micrometers, less than 150 micrometers, less than 100 micrometers, less than 50 micrometers, or less than 25 micrometers. In some embodiments, the average particle size is 25 microns to 500 microns, e.g., 50 microns to 500 microns, 100 microns to 300 microns, 100 microns to 250 microns, 200 microns to 300 microns, or 200 microns to 250 microns.

In some such compositions and products, the nicotine-polymer complex is the sole nicotine component within the composition and/or product. In some embodiments, the nicotine-polymer complex is included in the composition or product along with one or more other types of nicotine components. By "nicotine component" is meant any suitable form of nicotine (e.g., freebase or protonated nicotine (e.g., ion-pair nicotine, ion-pair nicotine complex or nicotine salt) for providing oral absorption of at least a portion of the nicotine present. Various nicotinic compounds, and methods for their administration, are set forth in US Pat. Pub. No. 2011/0274628 to Borschke, which is incorporated herein by reference. As used herein, "nicotinic compound" often refers to naturally occurring or synthetic nicotinic compound unbound from a plant material, meaning the compound is at least partially purified and not contained within a plant structure, such as a tobacco leaf.

In some embodiments, a second (and/or third, fourth, fifth, etc., where applicable) nicotine component is selected from the group consisting of unbound nicotine, in the form of nicotine freebase or a nicotine salt. By "unbound nicotine" is meant that the nicotine is not intentionally added to the composition in the form of nicotine bound within a resin. The nicotine freebase or nicotine salt can be employed alone, *i.e.,* in the form of an extract or other purified material. In some embodiments, the nicotine freebase or nicotine salt can be adsorbed in for example, a microcrystalline cellulose material to form a microcrystalline cellulose-nicotine carrier complex. In some embodiments, the optional second (and/or third, fourth, fifth, etc.) nicotine component comprises a nicotine-polymer complex, which can be a conventional (e.g., commercially available nicotine-polymer complex) or can be prepared and provided according to the processes outlined herein.

The nicotine, in some embodiments, is naturally occurring and obtained as an extract from a *Nicotiana* species (e.g., tobacco). The nicotine can be, for example, in the form of a highly purified tobacco extract. Various methods are known for the isolation and purification of nicotine from tobacco (including, but not limited to, extraction from tobacco with water; extraction from tobacco with organic solvents; steam distillation from tobacco; or pyrolytic degradation of tobacco and distillation of nicotine therefrom). For exemplary extraction methods, see for example, U.S. Patent Nos. 2,822,306 and 4,153,063 to Roselius *et al.* and US Pat. App. Pub. No. 2008/0302377 to Kauryzbaev et al.*,* which are incorporated herein by reference. In some embodiments, nicotine may be obtained from another source (e.g., another type of plant).

In some embodiments, nicotine may be synthetically made. The method by which synthetic nicotine used in some embodiments of the compositions and products described herein is synthesized can vary and is not particularly limited. Various methods for the preparation of nicotine are known. *See, e.g.,* Florence L. Wagner et al., 63 Tetrahedron 8065 (2007); U.S. Patent No. 10,913,962 to McCague et al.; and U.S. Patent App. Pub. No. 2020/0331884 to Weber et al., which are incorporated herein by reference in their entireties.

The nicotine can have the enantiomeric form S(-)-nicotine, R(+)-nicotine, or a mixture of S(-)-nicotine and R(+)-nicotine. In some embodiments, the nicotine is in the form of S(-)-nicotine (e.g., in a form that is virtually all S(-)-nicotine) or a racemic mixture composed primarily or predominantly of S(-)-nicotine (e.g., a mixture composed of 95 weight parts S(-)-nicotine and 5 weight parts R(+)-nicotine). In some embodiments, the nicotine is employed in virtually pure form or in an essentially pure form. Example nicotine that is employed has a purity of greater than 95 percent, greater than 98 percent, or greater than 99 percent, on a weight basis.

The amount of the optional additional nicotine component(s), where included, can vary and may depend, in part, on the exact form of the nicotine component(s) (e.g., nicotine freebase or protonated nicotine). In some embodiments, the amount of nicotine provided by the optional additional nicotine component(s) where included can vary from 0%, 5%, 10%, 20%, 30%, 40%, 50%, or 60% to 40%, 50%, 60%, 70%, 80%, 90%, 95%, or 98%, based on the total weight of all nicotine components within a given composition. Certain, non-limiting example amounts of the optional additional nicotine component(s) incorporated within a given composition can range, e.g., from 0.01% by weight to 10% by weight, e.g., 0.1 to 1.5% by weight, 0.5% by weight to 5% by weight, 1% to 5% by weight, or 0.5% to 3% by weight, based on the total weight of the composition to be included within a pouched product.

The weight ratio of the nicotine-polymer complex to the additional nicotine component(s) (where present) in the compositions and products provided herein can vary. In some embodiments, the compositions and products comprise nicotine wholly or primarily in the form of the nicotine-polymer complex; in some embodiments, the compositions and products comprise nicotine primarily in the form of the optional additional nicotine component(s). In some embodiments, the nicotine-polymer complex and the optional additional nicotine component(s) are provided in roughly equivalent amounts by weight. Certain examples include, but are not limited to, compositions wherein 100% of the nicotine is in the form of a nicotine-polymer complex as described herein, compositions wherein 50% of the nicotine is in the form of a nicotine-polymer complex as described herein and 50% of the nicotine is in the form of nicotine freebase or protonated nicotine; and compositions wherein 80% of the nicotine is in the form of a nicotine-polymer complex as described herein and 20% of the nicotine is in the form of nicotine freebase or protonated nicotine, as well as all ranges there between.

The total amount of nicotine provided by the nicotine component(s) provided herein can also vary. In some embodiments, a pouch is provided including a composition which comprises 5 mg to 25 mg nicotine, *e.g.,* 5 mg nicotine to 20 mg nicotine, and 10 mg nicotine to 15 mg nicotine. Certain, non-limiting examples of pouch compositions comprise 5 mg nicotine per pouch, 10 mg nicotine per pouch, 15 mg nicotine per pouch, or 20 mg nicotine per pouch. Some embodiments comprise 2% to 4% nicotine-polymer complex as described herein and 1% to 3% of an additional nicotine component; 3% to 5% nicotine-polymer complex as described herein and 1% to 3% of an additional nicotine component; and 8% to 10% of the nicotine-polymer complex as described herein and 1% to 3% of an additional nicotine component, all on a weight basis based on the entirety of the composition, *e.g.,* as included within a pouched product.

### Filler Component

The material within the pouches as described herein typically includes at least one particulate filler component. Such particulate filler components may fulfill multiple functions, such as enhancing certain organoleptic properties such as texture and mouthfeel, enhancing cohesiveness or compressibility of the product, and the like. Generally, the filler components are particulate materials and are cellulose-based. For example, suitable particulate filler components are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX^{®} brand filler available from International Fiber Corporation), bran fiber, and mixtures thereof. Non-limiting examples of derivatives of non-tobacco plant material include starches (e.g., from potato, wheat, rice, corn), natural cellulose, and modified cellulosic materials. Additional examples of potential particulate filler components include maltodextrin, dextrose, calcium carbonate, calcium phosphate, lactose, mannitol, xylitol, and sorbitol. Combinations of fillers can also be used.

"Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the mixture based on the ability of the starch material to impart a specific organoleptic property to composition. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications, and are considered to be "genetically modified" starches. Other starches are obtained and subsequently physically (e.g., heat, cool water swelling, etc.), chemically, or enzymatically modified. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, acetylation, hydroxypropylation, and/or partial hydrolysis. Enzymatic treatment includes subjecting native starches to enzyme isolates or concentrates, microbial enzymes, and/or enzymes native to plant materials, e.g., amylase present in corn kernels to modify corn starch. Other starches are modified by heat treatments, such as pregelatinization, dextrinization, and/or cold water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, starch sodium octenyl succinate.

In some embodiments, the filler comprises or is an inorganic material. Examples of potential inorganic fillers include calcium carbonate, calcium phosphate, and bioceramic materials (e.g., porous hydroxyapatite).

In some embodiments, the particulate filler component is a cellulose material or cellulose derivative. One particularly suitable particulate filler component for use in the products described herein is microcrystalline cellulose ("MCC"). The MCC may be synthetic or semisynthetic, or it may be obtained entirely from natural celluloses. The MCC may be selected from the group consisting of AVICEL^{®} grades PH-100, PH-102, PH-103, PH-105, PH-112, PH-113, PH-200, PH-300, PH-302, VIVACEL^{®} grades 101, 102, 12, 20 and EMOCEL^{®} grades 50M and 90M, and the like, and mixtures thereof. In some embodiments, the mixture comprises MCC as the particulate filler component. The quantity of MCC present in the mixture as described herein may vary according to the desired properties.

In some embodiments, a particulate filler can be characterized as substantially spherical, such as cellulose spheres. By "substantially spherical" is meant at least a portion of the particulate filler component is in the shape of a sphere and/or is "sphere-like' in shape. As such, "substantially spherical" encompasses slightly elongated (e.g., oval) shapes, slightly flattened shapes, and the like. Substantially spherical particulate filler components are intended to be distinguished from conventional particulate filler components (e.g., commercially available "fillers" or "particulate fillers" that are not explicitly designed as "spherical"). Such conventional particulate filler components typically substantially comprise particles with rather irregular shapes. In some cases, at least some (e.g., including a majority) of such conventional particulate filler components comprise one or more edges (e.g., jagged edges) that are typically not observable on substantially spherical particulate filler components as employed in the context of the present disclosure. Further, the uniformity of the particle shapes and sizes of a substantially spherical filler component is generally much greater than that of a conventional particulate filler component.

In some embodiments, the substantially spherical filler component comprises MCC. In some embodiments, the substantially spherical filler component comprises solid (although porous) MCC spheres. In some embodiments, the substantially spherical filler component comprises hollow MCC spheres. In some embodiments, the center/core of such hollow MCC spheres may be unfilled; in some embodiments, the center/core of such hollow MCC spheres may be filled with one or more additional components (e.g., flavorants, fillers, active ingredients, etc.). Examples of suitable MCC spheres include, but are not limited to, Vivapur^{®} MCC spheres from JRS Pharma, available, e.g., with particle sizes of 100-200 µm (Vivapur^{®} 100), 200-355 µm (Vivapur^{®} 200), 355-500 µm (Vivapur^{®} 350), 500-710 µm (Vivapur^{®} 500), 710-1000 µm (Vivapur^{®}700), and 1000-1400 µm (Vivapur^{®} 1000). Further examples of suitable MCC spheres include, but are not limited to, Celphere^{™} MCC spheres from Asahi Kasei Corporation, available, e.g., with particle sizes of 75-212 µm (Celphere^{™} SCP-100), 106-212 µm (Celphere^{™} CP-102), 150-300 µm (Celphere^{™} CP-203), 300-500 µm (Celphere^{™} CP-305), and 500-710 µm (Celphere^{™} CP-507).

The average diameter of the substantially spherical particulate filler particles provided herein can vary, and is not particularly limited. For example, in some embodiments, the spherical filler particles have an average diameter of 100 microns to 1000 microns, such as 250 microns to 750 microns. For example, in some embodiments, the average diameter is 100 microns to 500 microns, e.g., 100 microns to 400 microns, 100 microns to 300 microns, 100 microns to 200 microns, 200 microns to 500 microns, 200 microns to 400 microns, 200 microns to 300 microns, 300 microns to 500 microns, 300 microns to 400 microns, or 400 microns to 500 microns. In some embodiments, the average diameter is 500 microns to 1000 microns, e.g., 500 microns to 900 microns, 500 microns to 800 microns, 500 microns to 700 microns, 500 microns to 600 microns, 600 microns to 1000 microns, 600 microns to 900 microns, 600 microns to 800 microns, 600 microns to 700 microns, 700 microns to 1000 microns, 700 microns to 900 microns, 700 microns to 800 microns, 800 microns to 1000 microns, 800 microns to 900 microns, or 900 microns to 1000 microns.

The distribution of diameters around this average diameter (i.e., the particle size distribution) can also vary; in some embodiments, the distribution of diameters is close to the listed value (e.g., +/- 25% of the stated value, +/- 20% of the stated value, +/- 15% of the stated value, +/- 10% of the stated value, +/- 5% of the stated value, or +/- 1% of the stated value. The disclosure is not, however, limited to materials with such narrow distributions; in some embodiments, the diameter of the MCC spheres within a given material can vary within a wider range.

The amount of particulate filler component can vary, but is typically up to 75 percent of the material contained within the pouch by weight (i.e., the mixture), based on the total weight of the mixture. A typical range of particulate filler material (e.g., MCC) within the mixture can be from 10 to 75 percent by total weight of the mixture, for example, from 10, 15, 20, 25, or 30, to 35, 40, 45, or 50 weight percent (e.g., 20 to 50 weight percent, 25 to 45 weight percent, or 50 to 80 weight percent or 60 to 80 weight percent). In some embodiments, the amount of particulate filler material is at least 10 percent by weight, such as at least 20 percent, or at least 25 percent, or at least 30 percent, or at least 35 percent, or at least 40 percent, based on the total weight of the mixture.

In some embodiments, the particulate filler component further comprises a cellulose derivative or a combination of such derivatives. In some embodiments, the mixture comprises from 1 to 10% of the cellulose derivative by weight, based on the total weight of the mixture, with some embodiments comprising 1 to 5% by weight of cellulose derivative. In some embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In some embodiments, the cellulose derivative is one or more of methylcellulose, HPC, HPMC, hydroxyethyl cellulose, and CMC. In some embodiments, the cellulose derivative is HPC. In some embodiments, the mixture comprises from 0% to 5% HPC by weight, e.g., 1% to 3% HPC by weight, based on the total weight of the mixture.

In some embodiments, the composition comprises, as a filler, a byproduct of a pulping process, such as citrus rinds. In some embodiments, the composition comprises, as a filler, wheat straw. Such fillers can be used in combination with any of the types of particulate fillers referenced herein above.

### Water

The water content of the mixture within the pouched product described herein, prior to use by a consumer of the product, may vary according to the desired properties. Typically, the mixture, as present within the product prior to insertion into the mouth of the user, is less than 60 percent by weight of water, and generally is from 1% to 60% by weight of water, for example, from 5% to 55%, 10% to 50%, 20% to 45%, or 25% to 40% water by weight, including water amounts of at least 5% by weight, at least 10% by weight, at least 15% by weight, and at least 20% by weight.

In some embodiments, the mixture comprises a lower water content than some conventional mixtures for inclusion within a pouched product. For example, in some embodiments, the mixture comprises water in an amount of up to 25% by weight or up to 20% by weight, based on the total weight of the mixture. In some embodiments, the water content of the particulate composition is 1% to 12% by weight, such as less than 8%, less than 7%, less than 6%, less than 5%, or less than 4% by weight, based on the total weight of the particulate composition. Example embodiments can include water in an amount of 15% to 25%, e.g., 17% to 20%.

### Flavoring agent

As used herein, a "flavoring agent" or "flavorant" is any flavorful or aromatic substance capable of altering the sensory characteristics associated with the oral product. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy. Specific types of flavors include, but are not limited to, vanilla, coffee, chocolate/cocoa, cream, mint, spearmint, menthol, peppermint, wintergreen, eucalyptus, lavender, cardamon, nutmeg, cinnamon, clove, cascarilla, sandalwood, honey, jasmine, ginger, anise, sage, licorice, lemon, orange, apple, peach, lime, cherry, strawberry, trigeminal sensates, melatonin, terpenes, and any combinations thereof. See also, Leffingwell et al., Tobacco Flavoring for Smoking Products, R. J. Reynolds Tobacco Company (1972), which is incorporated herein by reference. Flavorings also may include components that are considered moistening, cooling or smoothening agents, such as eucalyptus. These flavors may be provided neat (i.e., alone) or in a composite, and may be employed as concentrates or flavor packages (e.g., spearmint and menthol, orange and cinnamon; lime, pineapple, and the like). Representative types of components also are set forth in US Pat. No. 5,387,416 to White et al.; US Pat. App. Pub. No. 2005/0244521 to Strickland et al.; and PCT Application Pub. No. WO 05/041699 to Quinter et al., each of which is incorporated herein by reference. In some instances, the flavoring agent may be provided in a spray-dried form or a liquid form.

The flavoring agent generally comprises at least one volatile flavor component. As used herein, "volatile" refers to a chemical substance that forms a vapor readily at ambient temperatures (i.e., a chemical substance that has a high vapor pressure at a given temperature relative to a nonvolatile substance). Typically, a volatile flavor component has a molecular weight below 400 Da, and often include at least one carbon-carbon double bond, carbon-oxygen double bond, or both. In some embodiments, the at least one volatile flavor component comprises one or more alcohols, aldehydes, aromatic hydrocarbons, ketones, esters, terpenes, terpenoids, or a combination thereof. Non-limiting examples of aldehydes include vanillin, ethyl vanillin, p-anisaldehyde, hexanal, furfural, isovaleraldehyde, cuminaldehyde, benzaldehyde, and citronellal. Non-limiting examples of ketones include 1-hydroxy-2-propanone and 2-hydroxy-3-methyl-2-cyclopentenone-1-one. Non-limiting examples of esters include allyl hexanoate, ethyl heptanoate, ethyl hexanoate, isoamyl acetate, and 3-methylbutyl acetate. Non-limiting examples of terpenes include sabinene, limonene, gamma-terpinene, beta-farnesene, nerolidol, thujone, myrcene, geraniol, nerol, citronellol, linalool, and eucalyptol. In some embodiments, the at least one volatile flavor component comprises one or more of ethyl vanillin, cinnamaldehyde, sabinene, limonene, gamma-terpinene, beta-farnesene, or citral. In some embodiments, the at least one volatile flavor component comprises ethyl vanillin.

The amount of flavoring agent utilized in the mixture can vary, but is typically up to 10 weight percent, and some embodiments are characterized by a flavoring agent content of at least 0.1 weight percent, such as 0.5 to 10 weight percent, 1 to 6 weight percent, or 2 to 5 weight percent, based on the total weight of the composition.

The amount of flavoring agent present within the mixture may vary over a period of time (e.g., during a period of storage after preparation of the mixture). For example, certain volatile components present in the mixture may evaporate or undergo chemical transformations, leading to a reduction in the concentration of one or more volatile flavor components. In some embodiments, a concentration of one or more of the at least one volatile flavor components present is greater than a concentration of the same one or more volatile flavor components present in a control pouched product which does not include the one or more organic acids, after the same time period. Without wishing to be bound by theory, it is believed that the same mechanisms responsible for loss of whiteness result in a gradual decline in certain volatile components in the flavoring (e.g., aldehydes, ketones, terpenes). Therefore, a decline in the presence of these volatile components leading to the discoloration over time may be expected to diminish the sensory satisfaction associated with products subject to such a degradation process.

### Salt

In some embodiments, the mixture may further comprise a salt (e.g., alkali metal salts), typically employed in an amount sufficient to provide desired sensory attributes to the mixture. Non-limiting examples of suitable salts include sodium chloride, potassium chloride, ammonium chloride, flour salt, and the like. When present, a representative amount of salt is 0.5 percent by weight or more, 1.0 percent by weight or more, or at 1.5 percent by weight or more, but will typically make up 10 percent or less of the total weight of the mixture, or 7.5 percent or less or 5 percent or less (e.g., 0.5 to 5 percent by weight).

### Sweetener

The mixture typically further comprises one or more sweeteners. The sweeteners can be any sweetener or combination of sweeteners, in natural or artificial form, or as a combination of natural and artificial sweeteners. Examples of natural sweeteners include isomaltulose, fructose, sucrose, glucose, maltose, mannose, galactose, lactose, stevia, honey, and the like. Examples of artificial sweeteners include sucralose, maltodextrin, saccharin, aspartame, acesulfame K, neotame and the like. In some embodiments, the sweetener comprises one or more sugar alcohols. Sugar alcohols are polyols derived from monosaccharides or disaccharides that have a partially or fully hydrogenated form. Sugar alcohols have, for example, 4 to 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). When present, a representative amount of sweetener may make up from 0.1 to 20 percent or more of the of the mixture by weight, for example, from 0.1% to 1%, from 1% to 5%, from 5% to 10%, or from 10% to 20% of the mixture on a weight basis, based on the total weight of the mixture.

### Binding agent

A binder (or combination of binders) may be employed in some embodiments, in amounts sufficient to provide the desired physical attributes and physical integrity to the mixture. Binders also often function as thickening or gelling agents. Typical binders can be organic or inorganic, or a combination thereof. Representative binders include modified cellulose, povidone, sodium alginate, starch-based binders, pectin, carrageenan, pullulan, zein, and the like, and combinations thereof. In some embodiments, the binder comprises pectin or carrageenan or combinations thereof.

A binder may be employed in amounts sufficient to provide the desired physical attributes and physical integrity to the mixture. The amount of binder utilized in the mixture can vary, but is typically up to 30 weight percent, and some embodiments are characterized by a binder content of at least 0.1% by weight, such as 1% to 30% by weight, or 5% to 10% by weight, based on the total weight of the mixture.

In some embodiments, the binder includes a gum, for example, a natural gum. As used herein, a natural gum refers to polysaccharide materials of natural origin that have binding properties, and which are also useful as a thickening or gelling agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. When present, natural gum binder materials are typically present in an amount of up to 5% by weight, for example, from 0.1%, 0.2%, 0.3%, 0.4%, 0.5%, 0.6%, 0.7%, 0.8%, 0.9%, or 1%, to 2%, 3%, 4%, or 5% by weight, based on the total weight of the mixture.

### Humectant

In some embodiments, one or more humectants may be employed in the mixture. Examples of humectants include, but are not limited to, glycerin, propylene glycol, and the like. Where included, the humectant is typically provided in an amount sufficient to provide desired moisture attributes to the mixture. Further, in some instances, the humectant may impart desirable flow characteristics to the mixture for depositing in a mold. When present, a humectant will typically make up 10% or less of the weight of the mixture (e.g., from 0.5% to 8% by weight). When present, a representative amount of humectant is 0.1% to 1% by weight, 0.1% to 0.5% by weight, 1% to 5% by weight, 2% to 10% by weight, or 5% to 10% by weight based on the total weight of the mixture. In some embodiments, a humectant (e.g., glycerol) can improve the flavor release and/or flavor intensity profile of the disclosed products.

### pH Adjuster/Buffering Agent

In some embodiments, the disclosed oral compositions comprise a base, in some embodiments, a relatively strong base to release the nicotine from the nicotine-polymer complex that may be present within the composition. For example, in some embodiments a pH adjuster that is a stronger base than a carbonate is included within the composition. Examples of bases that may be sufficient for this purpose include, but are not limited to, metal hydroxides such as potassium hydroxide, calcium hydroxide, and sodium hydroxide. It is noted that, in some embodiments, additional buffering agents/pH adjusters may be included in the disclosed compositions (e.g., including, but not limited to, metal carbonates); such additional components can, in some embodiments, serve one or more additional functions within the composition (e.g., modifying the flavor of the composition). Examples of pH adjusters and buffering agents that can be used include, but are not limited to, metal hydroxides (e.g., alkali metal hydroxides such as sodium hydroxide, and potassium hydroxide) and alkaline earth metal hydroxides (e.g., calcium hydroxide and magnesium hydroxide), as well as other alkali metal buffers such as metal carbonates (e.g., potassium carbonate, calcium carbonate, or sodium carbonate), or metal bicarbonates such as sodium bicarbonate, potassium bicarbonate, and the like. Additional, non-limiting examples include ammonia hydroxide, potassium acetate, sodium acetate, sodium benzoate, sodium sesquicarbonate, trisodium phosphate, and combinations thereof. In some embodiments, calcium hydroxide is particularly useful as a base/pH adjuster.

In some embodiments, the pH of compositions and products within the scope of the present disclosure is considered basic, *i.e.,* having a pH greater than 7 (e.g., greater than 7.5, greater than 8, or greater than 8.5). In some embodiments, the pH of the composition and/or product is 7 to 10, e.g., 7 to 9.5, 7 to 9, 8 to 10, 8.5 to 9.5, 8.5 to 9, 8.6 to 9.2, or 8.6 to 9.0. In some embodiments, the pH of compositions and products within the scope of the present disclosure is greater than 6.5, e.g., 6.5 to 10, 6.5 to 9, or 6.5 to 8. In some embodiments, the pH of compositions and products within the scope of the present disclosure is 7 or below, e.g., 6.5 or below. pH measurements can be conducted, e.g., by placing 1.5 g of the composition to be tested (which can be, e.g., two pouches) in 30 mL of water. A stir bar magnet is placed inside the container and the mixture is stirred while conducting the pH measurement using a pH meter with glass electrode.

Where present, a buffering agent is typically present in an amount less than 5 percent based on the weight of the mixture, for example, from 0.1% to 1%, 0.1% to 0.5%, or 0.5% to 5%, such as, *e.g.,* from 0.75% to 4%, from 0.75% to 3%, or from 1% to 2% by weight, based on the total weight of the composition. Non-limiting examples of suitable buffers include alkali metals acetates, glycinates, phosphates, glycerophosphates, citrates, carbonates, hydrogen carbonates, borates, or mixtures thereof.

### Colorant

A colorant may be employed in amounts sufficient to provide the desired physical attributes to the mixture Natural or synthetic colorants, such as natural or synthetic dyes, food-grade colorants and pharmaceutical-grade colorants may be used. Examples of colorants include various dyes and pigments, such as caramel coloring and titanium dioxide. Natural colorants such as curcumin, beet juice extract, spirulina; also a variety of synthetic pigments may also be used. In some embodiments, the colorant is a lake dye, such as a red or blue aluminum lake dye. The amount of colorant utilized in the oral composition can vary, but when present is typically up to 3% by weight, such as from 0.1%, 0.5%, or 1%, to 3% by weight, based on the total weight of the oral composition.

### Active ingredient

In some embodiments, the active ingredient contained within the disclosed oral products is nicotine (i.e., the referenced nicotine component or the first and second nicotine components). However, in some embodiments, the composition as disclosed herein includes one or more active ingredients in addition to such nicotine component(s).

As used herein, an "active ingredient" refers to one or more substances belonging to any of the following categories: API (active pharmaceutical ingredient), food additives, natural medicaments, and naturally occurring substances that can have an effect on humans. Example active ingredients that can be used in addition to the nicotine component(s) include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body). In some embodiments, the optional additional active ingredient may be of the type generally referred to as dietary supplements, nutraceuticals, "phytochemicals" or "functional foods." These types of additives are sometimes defined in the art as encompassing substances typically available from naturally-occurring sources (e.g., botanical materials) that provide one or more advantageous biological effects (e.g., health promotion, disease prevention, or other medicinal properties), but are not classified or regulated as drugs.

Non-limiting examples of additional active ingredients include those falling in the categories of botanical ingredients, stimulants, amino acids, and/or pharmaceutical, nutraceutical, and medicinal ingredients (e.g., vitamins, such as A, B3, B6, B12, and C, and/or cannabinoids, such as tetrahydrocannabinol (THC) and cannabidiol (CBD)). Each of these categories is further described herein below. The particular choice of additional active ingredients, where used, will vary depending upon the desired flavor, texture, and desired characteristics of the particular product.

In some embodiments, the additional active ingredient is selected from the group consisting of caffeine, taurine, GABA, theanine, vitamin C, lemon balm extract, ginseng, citicoline, sunflower lecithin, and combinations thereof. For example, the active ingredient can include a combination of caffeine, theanine, and optionally ginseng. In another embodiment, the active ingredient includes a combination of theanine, gamma-amino butyric acid (GABA), and lemon balm extract. In a further embodiment, the active ingredient includes theanine, theanine and tryptophan, or theanine and one or more B vitamins (e.g., vitamin B6 or B12). In a still further embodiment, the active ingredient includes a combination of caffeine, taurine, and vitamin C.

The particular percentages of additional active ingredients present will vary depending upon the desired characteristics of the particular product. Typically, such additional active ingredient or combination thereof, where present, is included in a total concentration of at least 0.001% by weight of the composition, such as in a range from 0.001% to 20%. In some embodiments, the optional additional active ingredient or combination of active ingredients, where present, is present in a concentration from 0.1% w/w to 10% by weight, such as, e.g., from 0.5% w/w to 10%, from 1% to 10%, from 1% to 5% by weight, based on the total weight of the composition. In some embodiments, the additional active ingredient or combination of active ingredients is included, where present, in a concentration of from 0.001%, 0.01%, 0.1% , or 1%, up to 20% by weight, such as, e.g., from 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5% 0.6%, 0.7%, 0.8%, or 0.9%, to 1%, 2%, 3%, 4%, 5%, 6%, 7%, 8%, 9%, 10%, 11%, 12%, 13%, 14%, 15%, 16%, 17%, 18%, 19%, or 20% by weight, based on the total weight of the composition. Further suitable ranges for specific active ingredients that can, in some embodiments, be employed in combination with the nicotine component(s) referenced above, are provided herein below.

### Botanical

In some embodiments, the active ingredient comprises a botanical ingredient. As used herein, the term "botanical ingredient" or "botanical" refers to any plant material or fungal-derived material, including plant material in its natural form and plant material derived from natural plant materials, such as extracts or isolates from plant materials or treated plant materials (e.g., plant materials subjected to heat treatment, fermentation, bleaching, or other treatment processes capable of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). Reference to botanical material as "non-tobacco" is intended to exclude tobacco materials (i.e., does not include any *Nicotiana* species). In some embodiments, the compositions and/or products as disclosed herein can be characterized as free of any tobacco material (e.g., any embodiment as disclosed herein may be completely or substantially free of any tobacco material). By "substantially free" is meant that no tobacco material has been intentionally added. For example, some embodiments can be characterized as having less than 1% by weight of tobacco, less than 0.5% by weight of tobacco, less than 0.1% by weight of tobacco, less than 0.01% by weight of tobacco, less than 0.001% by weight of tobacco, or less than 0.0001%, or even 0% by weight of tobacco.

When present, a botanical is typically at a concentration of from 0.01% w/w to 10% by weight, such as, e.g., from 0.01% w/w, 0.05%, 0.1%, or 0.5%, to 1%, 2%, 3%, 4%, 5%, 6%, 7%, 8%, 9%, or 10%, 11%, 12%, 13%, 14%, or 15% by weight, based on the total weight of the composition.

The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein. Non-limiting examples of botanicals or botanical-derived materials include ashwagandha, *Bacopa monniera,* baobab, basil, *Centella asiatica,* Chai-hu, chamomile, cherry blossom, chlorophyll, cinnamon, citrus, cloves, cocoa, cordyceps, curcumin, damiana, *Dorstenia arifolia, Dorstenia odorata,* essential oils, eucalyptus, fennel, *Galphimia glauca,* ginger, *Ginkgo biloba,* ginseng (e.g., *Panax ginseng*), green tea, *Griffonia simplicifolia,* guarana, cannabis, hemp, hops, jasmine, *Kaempferia parviflora* (Thai ginseng), kava, lavender, lemon balm, lemongrass, licorice, lutein, maca, matcha, Nardostachys chinensis, oil-based extract of *Viola odorata,* peppermint, quercetin, resveratrol, *Rhizoma gastrodiae, Rhodiola, rooibos,* rose essential oil, rosemary, *Sceletium tortuosum,* Schisandra, Skullcap, spearmint extract, Spikenard, terpenes, tisanes, turmeric, *Turnera aphrodisiaca,* valerian, white mulberry, and *Yerba mate.*

In some embodiments, the active ingredient comprises lemon balm. Lemon balm (*Melissa officinalis*) is a mildly lemon-scented herb from the same family as mint (*Lamiaceae*). The herb is native to Europe, North Africa, and West Asia. The tea of lemon balm, as well as the essential oil and the extract, are used in traditional and alternative medicine. In some embodiments, the active ingredient comprises lemon balm extract. In some embodiments, the lemon balm extract is present in an amount of from 1 to 4% by weight, based on the total weight of the composition.

In some embodiments, the active ingredient comprises ginseng. Ginseng is the root of plants of the genus *Panax,* which are characterized by the presence of unique steroid saponin phytochemicals (ginsenosides) and gintonin. Ginseng finds use as a dietary supplement in energy drinks or herbal teas, and in traditional medicine. Cultivated species include Korean ginseng (*P. ginseng*), South China ginseng (*P. notoginseng*), and American ginseng (*P. quinquefolius*). American ginseng and Korean ginseng vary in the type and quantity of various ginsenosides present. In some embodiments, the ginseng is American ginseng or Korean ginseng. In some embodiments, the active ingredient comprises Korean ginseng. In some embodiments, ginseng is present in an amount of from 0.4% to 0.6% by weight, based on the total weight of the composition.

### Stimulant

In some embodiments, the active ingredient comprises one or more stimulants. As used herein, the term "stimulant" refers to a material that increases activity of the central nervous system and/or the body, for example, enhancing focus, cognition, vigor, mood, alertness, and the like. Non-limiting examples of stimulants include caffeine, theacrine, theobromine, and theophylline. Theacrine (1,3,7,9-tetramethyluric acid) is a purine alkaloid which is structurally related to caffeine, and possesses stimulant, analgesic, and anti-inflammatory effects. Present stimulants may be natural, naturally derived, or wholly synthetic. For example, certain botanical materials (guarana, tea, coffee, cocoa, and the like) may possess a stimulant effect by virtue of the presence of e.g., caffeine or related alkaloids, and accordingly are "natural" stimulants. By "naturally derived" is meant the stimulant (e.g., caffeine, theacrine) is in a purified form, outside its natural (e.g., botanical) matrix. For example, caffeine can be obtained by extraction and purification from botanical sources (e.g., tea). By "wholly synthetic", it is meant that the stimulant has been obtained by chemical synthesis. In some embodiments, the active ingredient comprises caffeine. In some embodiments, the caffeine is present in an encapsulated form. On example of an encapsulated caffeine is Vitashure^{®}, available from Balchem Corp., 52 Sunrise Park Road, New Hampton, NY, 10958.

When present, a stimulant or combination of stimulants (e.g., caffeine, theacrine, and combinations thereof) is typically at a concentration of from 0.1% w/w to 15% by weight, such as, e.g., from 0.1% w/w, 0.2%, 0.3%, 0.4%, 0.5% 0.6%, 0.7%, 0.8%, or 0.9%, to 1%, 2%, 3%, 4%, 5%, 6%, 7%, 8%, 9%, 10%, 11%, 12%, 13%, 14%, or 15% by weight, based on the total weight of the composition. In some embodiments, the composition comprises caffeine in an amount of from 1.5 to 6% by weight, based on the total weight of the composition.

### Amino acid

In some embodiments, the active ingredient comprises an amino acid. As used herein, the term "amino acid" refers to an organic compound that contains amine (-NH₂) and carboxyl (-COOH) or sulfonic acid (SO₃H) functional groups, along with a side chain (R group), which is specific to each amino acid. Amino acids may be proteinogenic or non-proteinogenic. By "proteinogenic" is meant that the amino acid is one of the twenty naturally occurring amino acids found in proteins. The proteinogenic amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. By "non-proteinogenic" is meant that either the amino acid is not found naturally in protein, or is not directly produced by cellular machinery (e.g., is the product of post-translational modification). Non-limiting examples of non-proteinogenic amino acids include gamma-aminobutyric acid (GABA), taurine (2-aminoethanesulfonic acid), theanine (L-γ-glutamylethylamide), hydroxyproline, and beta-alanine. In some embodiments, the active ingredient comprises theanine. In some embodiments, the active ingredient comprises GABA. In some embodiments, the active ingredient comprises a combination of theanine and GABA. In some embodiments, the active ingredient is a combination of theanine, GABA, and lemon balm. In some embodiments, the active ingredient is a combination of caffeine, theanine, and ginseng. In some embodiments, the active ingredient comprises taurine. In some embodiments, the active ingredient is a combination of caffeine and taurine.

When present, an amino acid or combination of amino acids (e.g., theanine, GABA, and combinations thereof) is typically at a concentration of from 0.1% w/w to 15% by weight, such as, e.g., from 0.1% w/w, 0.2%, 0.3%, 0.4%, 0.5% 0.6%, 0.7%, 0.8%, or 0.9%, to 1%, 2%, 3%, 4%, 5%, 6%, 7%, 8%, 9%, 10%, 11%, 12%, 13%, 14%, or 15% by weight, based on the total weight of the composition.

### Vitamins

In some embodiments, the active ingredient comprises a vitamin or combination of vitamins. As used herein, the term "vitamin" refers to an organic molecule (or related set of molecules) that is an essential micronutrient needed for the proper functioning of metabolism in a mammal. There are thirteen vitamins required by human metabolism, which are: vitamin A (as all-trans-retinol, all-trans-retinyl-esters, as well as all-trans-beta-carotene and other provitamin A carotenoids), vitamin B1 (thiamine), vitamin B2 (riboflavin), vitamin B3 (niacin), vitamin B5 (pantothenic acid), vitamin B6 (pyridoxine), vitamin B7 (biotin), vitamin B9 (folic acid or folate), vitamin B12 (cobalamins), vitamin C (ascorbic acid), vitamin D (calciferols), vitamin E (tocopherols and tocotrienols), and vitamin K (quinones). In some embodiments, the active ingredient comprises vitamin C. In some embodiments, the active ingredient is a combination of vitamin C, caffeine, and taurine.

When present, a vitamin or combination of vitamins (e.g., vitamin B6, vitamin B12, vitamin E, vitamin C, or a combination thereof) is typically at a concentration of from 0.01% w/w to 6% by weight, such as, e.g., from 0.01%, 0.02%, 0.03%, 0.04%, 0.05%, 0.06%, 0.07%, 0.08%, 0.09%, or 0.1% w/w, to 0.2%, 0.3%, 0.4%, 0.5% 0.6%, 0.7%, 0.8%, 0.9%, 1%, 2%, 3%, 4%, 5% , or 6% by weight, based on the total weight of the composition.

### Antioxidants

In some embodiments, the active ingredient comprises one or more antioxidants. As used herein, the term "antioxidant" refers to a substance which prevents or suppresses oxidation by terminating free radical reactions and may delay or prevent some types of cellular damage. Antioxidants may be naturally occurring or synthetic. Naturally occurring antioxidants include those found in foods and botanical materials. Non-limiting examples of antioxidants include certain botanical materials, vitamins, polyphenols, and phenol derivatives.

Examples of botanical materials which are associated with antioxidant characteristics include without limitation acai berry, alfalfa, allspice, annatto seed, apricot oil, basil, bee balm, wild bergamot, black pepper, blueberries, borage seed oil, bugleweed, cacao, calamus root, catnip, catuaba, cayenne pepper, chaga mushroom, chervil, cinnamon, dark chocolate, potato peel, grape seed, ginseng, gingko biloba, Saint John's Wort, saw palmetto, green tea, black tea, black cohosh, cayenne, chamomile, cloves, cocoa powder, cranberry, dandelion, grapefruit, honeybush, echinacea, garlic, evening primrose, feverfew, ginger, goldenseal, hawthorn, hibiscus flower, jiaogulan, kava, lavender, licorice, marjoram, milk thistle, mints (menthe), oolong tea, beet root, orange, oregano, papaya, pennyroyal, peppermint, red clover, rooibos (red or green), rosehip, rosemary, sage, clary sage, savory, spearmint, spirulina, slippery elm bark, sorghum bran hi-tannin, sorghum grain hi-tannin, sumac bran, comfrey leaf and root, goji berries, gutu kola, thyme, turmeric, uva ursi, valerian, wild yam root, wintergreen, yacon root, yellow dock, yerba mate, yerba santa, bacopa monniera, withania somnifera, Lion's mane, and silybum marianum. Such botanical materials may be provided in fresh or dry form, essential oils, or may be in the form of an extracts. The botanical materials (as well as their extracts) often include compounds from various classes known to provide antioxidant effects, such as minerals, vitamins, isoflavones, phytoesterols, allyl sulfides, dithiolthiones, isothiocyanates, indoles, lignans, flavonoids, polyphenols, and carotenoids. Examples of compounds found in botanical extracts or oils include ascorbic acid, peanut endocarb, resveratrol, sulforaphane, beta-carotene, lycopene, lutein, co-enzyme Q, carnitine, quercetin, kaempferol, and the like. See, e.g., Santhosh et al., Phytomedicine, 12(2005) 216-220, which is incorporated herein by reference.

Non-limiting examples of other suitable antioxidants include citric acid, Vitamin E or a derivative thereof, a tocopherol, epicatechol, epigallocatechol, epigallocatechol gallate, erythorbic acid, sodium erythorbate, 4-hexylresorcinol, theaflavin, theaflavin monogallate A or B, theaflavin digallate, phenolic acids, glycosides, quercitrin, isoquercitrin, hyperoside, polyphenols, catechols, resveratrols, oleuropein, butylated hydroxyanisole (BHA), butylated hydroxytoluene (BHT), tertiary butylhydroquinone (TBHQ), and combinations thereof.

When present, an antioxidant is typically at a concentration of from 0.001% w/w to 10% by weight, such as, e.g., from 0.001%, 0.005%, 0.01% w/w, 0.05%, 0.1%, or 0.5%, to 1%, 2%, 3%, 4%, 5%, 6%, 7%, 8%, 9%, or 10%, based on the total weight of the composition.

### Cannabinoids

In some embodiments, the active ingredient comprises one or more cannabinoids. As used herein, the term "cannabinoid" refers to a class of diverse chemical compounds that acts on cannabinoid receptors, also known as the endocannabinoid system, in cells that alter neurotransmitter release in the brain. Ligands for these receptor proteins include the endocannabinoids produced naturally in the body by animals; phytocannabinoids, found in cannabis; and synthetic cannabinoids, manufactured artificially. Cannabinoids found in cannabis include, without limitation: cannabigerol (CBG), cannabichromene (CBC), cannabidiol (CBD), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinol propyl variant (CBNV), cannabitriol (CBO), tetrahydrocannabinolic acid (THCA), and tetrahydrocannabivarinic acid (THCV A). In some embodiments, the cannabinoid is selected from tetrahydrocannabinol (THC), the primary psychoactive compound in cannabis, and cannabidiol (CBD) another major constituent of the plant, but which is devoid of psychoactivity. Each of the above compounds can be used in the form of an isolate from plant material or synthetically derived.

Alternatively, the active ingredient can be a cannabimimetic, which is a class of compounds derived from plants other than cannabis that have biological effects on the endocannabinoid system similar to cannabinoids. Examples include yangonin, alpha-amyrin or beta-amyrin (also classified as terpenes), cyanidin, curcumin (tumeric), catechin, quercetin, salvinorin A, N-acylethanolamines, and N-alkylamide lipids.

When present, a cannabinoid (e.g., CBD) or cannabimimetic is typically in a concentration of at least 0.1% by weight of the composition, such as in a range from 0.1% to 30%, such as, e.g., from 0.1%, 0.2%, 0.3%, 0.4%, 0.5% 0.6%, 0.7%, 0.8%, or 0.9%, to 1%, 2%, 3%, 4%, 5%, 6%, 7%, 8%, 9%, 10%, 15%, 20%, or 30% by weight, based on the total weight of the composition.

### Terpenes

Active ingredients suitable for use in the present disclosure can also be classified as terpenes, many of which are associated with biological effects, such as calming effects. Terpenes are understood to have the general formula of (C₅H₈)ₙ and include monoterpenes, sesquiterpenes, and diterpenes. Terpenes can be acyclic, monocyclic or bicyclic in structure. Some terpenes provide an entourage effect when used in combination with cannabinoids or cannabimimetics. Examples include beta-caryophyllene, linalool, limonene, beta-citronellol, linalyl acetate, pinene (alpha or beta), geraniol, carvone, eucalyptol, menthone, iso-menthone, piperitone, myrcene, beta-bourbonene, and germacrene, which may be used singly or in combination.

### Pharmaceutical ingredients

In some embodiments, the active ingredient comprises an active pharmaceutical ingredient (API). The API can be any known agent adapted for therapeutic, prophylactic, or diagnostic use. These can include, for example, synthetic organic compounds, proteins and peptides, polysaccharides and other sugars, lipids, phospholipids, inorganic compounds (e.g., magnesium, selenium, zinc, nitrate), neurotransmitters or precursors thereof (e.g., serotonin, 5-hydroxytryptophan, oxitriptan, acetylcholine, dopamine, melatonin), and nucleic acid sequences, having therapeutic, prophylactic, or diagnostic activity. Non-limiting examples of APIs include analgesics and antipyretics (e.g., acetylsalicylic acid, acetaminophen, 3-(4-isobutylphenyl)propanoic acid), phosphatidylserine, myoinositol, docosahexaenoic acid (DHA, Omega-3), arachidonic acid (AA, Omega-6), S-adenosylmethionine (SAM), beta-hydroxy-beta-methylbutyrate (HMB), citicoline (cytidine-5'-diphosphate-choline), and cotinine. In some embodiments, the active ingredient comprises citicoline. In some embodiments, the active ingredient is a combination of citicoline, caffeine, theanine, and ginseng. In some embodiments, the active ingredient comprises sunflower lecithin. In some embodiments, the active ingredient is a combination of sunflower lecithin, caffeine, theanine, and ginseng.

The amount of API may vary. For example, when present, an API is typically at a concentration of from 0.001% w/w to 10% by weight, such as, e.g., from 0.01%, 0.02%, 0.03%, 0.04%, 0.05%, 0.06%, 0.07%, 0.08%, 0.09%, 0.1% w/w, 0.2%, 0.3%, 0.4%, 0.5% 0.6%, 0.7%, 0.8%, 0.9%, or 1%, to 2%, 3%, 4%, 5%, 6%, 7%, 8%, 9%, or 10% by weight, based on the total weight of the composition.

In some embodiments, the composition is substantially free of any API. By "substantially free of any API" means that the composition does not contain, and specifically excludes, the presence of any API as defined herein, such as any Food and Drug Administration (FDA) approved therapeutic agent intended to treat any medical condition.

### Tobacco material

In some embodiments, the mixture may include a tobacco material. The tobacco material can vary in species, type, and form. Generally, the tobacco material is obtained from for a harvested plant of the *Nicotiana* species. Example *Nicotiana* species include N. tabacum, N. rustica, N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata, N. x sanderae, N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. simulans, N. stocktonii, N. suaveolens, N. umbratica, N. velutina, N. wigandioides, N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis subsp. Hersperis, N. pauciflora, N. petunioides, N. quadrivalvis, N. repanda, N. rotundifolia, N. solanifolia, and N. spegazzinii. Various representative other types of plants from the *Nicotiana* species are set forth in Goodspeed, *The Genus Nicotiana,* (Chonica Botanica) (1954); US Pat. Nos. 4,660,577 to Sensabaugh, Jr. et al.; 5,387,416 to White et al., 7,025,066 to Lawson et al.; 7,798,153 to Lawrence, Jr. and 8,186,360 to Marshall et al.; each of which is incorporated herein by reference. Descriptions of various types of tobaccos, growing practices and harvesting practices are set forth in Tobacco Production, Chemistry and Technology, Davis et al. (Eds.) (1999), which is incorporated herein by reference.

*Nicotiana* species from which suitable tobacco materials can be obtained can be derived using genetic-modification or crossbreeding techniques (e.g., tobacco plants can be genetically engineered or crossbred to increase or decrease production of components, characteristics or attributes). See, for example, the types of genetic modifications of plants set forth in US Pat. Nos. 5,539,093 to Fitzmaurice et al.; 5,668,295 to Wahab et al.; 5,705,624 to Fitzmaurice et al.; 5,844,119 to Weigl; 6,730,832 to Dominguez et al.; 7,173,170 to Liu et al.; 7,208,659 to Colliver et al. and 7,230,160 to Benning et al.; US Patent Appl. Pub. No. 2006/0236434 to Conkling et al.; and PCT WO2008/103935 to Nielsen et al. See, also, the types of tobaccos that are set forth in US Pat. Nos. 4,660,577 to Sensabaugh, Jr. et al.; 5,387,416 to White et al.; and 6,730,832 to Dominguez et al., each of which is incorporated herein by reference.

The *Nicotiana* species can, in some embodiments, be selected for the content of various compounds that are present therein. For example, plants can be selected on the basis that those plants produce relatively high quantities of one or more of the compounds desired to be isolated therefrom. In some embodiments, plants of the *Nicotiana* species (e.g., *Galpao commun* tobacco) are specifically grown for their abundance of leaf surface compounds. Tobacco plants can be grown in greenhouses, growth chambers, or outdoors in fields, or grown hydroponically.

Various parts or portions of the plant of the *Nicotiana* species can be included within a mixture as disclosed herein. For example, virtually all of the plant *(e.g.,* the whole plant) can be harvested, and employed as such. Alternatively, various parts or pieces of the plant can be harvested or separated for further use after harvest. For example, the flower, leaves, stem, stalk, roots, seeds, and various combinations thereof, can be isolated for further use or treatment. In some embodiments, the tobacco material comprises tobacco leaf (lamina). The mixture disclosed herein can include processed tobacco parts or pieces, cured and aged tobacco in essentially natural lamina and/or stem form, a tobacco extract, extracted tobacco pulp (e.g., using water as a solvent), or a mixture of the foregoing (e.g., a mixture that combines extracted tobacco pulp with granulated cured and aged natural tobacco lamina).

In some embodiments, the tobacco material comprises solid tobacco material selected from the group consisting of lamina and stems. The tobacco that is used for the mixture typically includes tobacco lamina, or a tobacco lamina and stem mixture (of which at least a portion is smoke-treated). Portions of the tobaccos within the mixture may have processed forms, such as processed tobacco stems (e.g., cut-rolled stems, cut-rolled-expanded stems or cut-puffed stems), or volume expanded tobacco (e.g., puffed tobacco, such as dry ice expanded tobacco (DIET)). See, for example, the tobacco expansion processes set forth in US Pat. Nos. 4,340,073 to de la Burde et al.; 5,259,403 to Guy et al.; and 5,908,032 to Poindexter, et al.; and 7,556,047 to Poindexter, et al., all of which are incorporated by reference. In addition, the d mixture optionally may incorporate tobacco that has been fermented. See, also, the types of tobacco processing techniques set forth in PCT WO2005/063060 to Atchley et al., which is incorporated herein by reference.

The tobacco material is typically used in a form that can be described as particulate (i.e., shredded, ground, granulated, or powder form). The manner by which the tobacco material is provided in a finely divided or powder type of form may vary. In some embodiments, plant parts or pieces are comminuted, ground or pulverized into a particulate form using equipment and techniques for grinding, milling, or the like. In some embodiments, the plant material is relatively dry in form during grinding or milling, using equipment such as hammer mills, cutter heads, air control mills, or the like. In some embodiments, the tobacco material is employed in the form of parts or pieces that have an average particle size between 1.4 millimeters and 250 microns. In some instances, the tobacco particles may be sized to pass through a screen mesh to obtain the particle size range of interest. If desired, air classification equipment may be used to ensure that small sized tobacco particles of the desired sizes, or range of sizes, may be collected. If desired, differently sized pieces of granulated tobacco may be mixed together.

The manner by which the tobacco is provided in a finely divided or powder type of form may vary. In some embodiments, tobacco parts or pieces are comminuted, ground or pulverized into a powder type of form using equipment and techniques for grinding, milling, or the like. In some embodiments, the tobacco is relatively dry in form during grinding or milling, using equipment such as hammer mills, cutter heads, air control mills, or the like. For example, tobacco parts or pieces may be ground or milled when the moisture content thereof is less than 15 weight percent to less than 5 weight percent. For example, the tobacco plant or portion thereof can be separated into individual parts or pieces (e.g., the leaves can be removed from the stems, and/or the stems and leaves can be removed from the stalk). The harvested plant or individual parts or pieces can be further subdivided into parts or pieces (e.g., the leaves can be shredded, cut, comminuted, pulverized, milled or ground into pieces or parts that can be characterized as filler-type pieces, granules, particulates or fine powders). The plant, or parts thereof, can be subjected to external forces or pressure (e.g., by being pressed or subjected to roll treatment). When carrying out such processing conditions, the plant or portion thereof can have a moisture content that approximates its natural moisture content (e.g., its moisture content immediately upon harvest), a moisture content achieved by adding moisture to the plant or portion thereof, or a moisture content that results from the drying of the plant or portion thereof. For example, powdered, pulverized, ground or milled pieces of plants or portions thereof can have moisture contents of less than 25 weight percent, often less than 20 weight percent, and frequently less than 15 weight percent.

For the preparation of oral products, it is typical for a harvested plant of the *Nicotiana* species to be subjected to a curing process. The tobacco materials incorporated within the mixture for inclusion within products as disclosed herein are those that have been appropriately cured and/or aged. Descriptions of various types of curing processes for various types of tobaccos are set forth in Tobacco Production, Chemistry and Technology, Davis et al. (Eds.) (1999). Examples of techniques and conditions for curing flue-cured tobacco are set forth in Nestor et al., Beitrage Tabakforsch. Int., 20, 467-475 (2003) and US Pat. No. 6,895,974 to Peele, which are incorporated herein by reference. Representative techniques and conditions for air curing tobacco are set forth in US Pat. No. 7,650,892 to Groves et al.; Roton et al., Beitrage Tabakforsch. Int., 21, 305-320 (2005) and Staaf et al., Beitrage Tabakforsch. Int., 21, 321-330 (2005), which are incorporated herein by reference. Certain types of tobaccos can be subjected to alternative types of curing processes, such as fire curing or sun curing.

In some embodiments, tobacco materials that can be employed include flue-cured or Virginia (e.g., K326), burley, sun-cured (e.g., Indian Kurnool and Oriental tobaccos, including Katerini, Prelip, Komotini, Xanthi and Yambol tobaccos), Maryland, dark, dark-fired, dark air cured (e.g., Madole, Passanda, Cubano, Jatin and Bezuki tobaccos), light air cured (e.g., North Wisconsin and Galpao tobaccos), Indian air cured, Red Russian and *Rustica* tobaccos, as well as various other rare or specialty tobaccos and various blends of any of the foregoing tobaccos.

The tobacco material may also have a so-called "blended" form. For example, the tobacco material may include a mixture of parts or pieces of flue-cured, burley (e.g., Malawi burley tobacco) and Oriental tobaccos (e.g., as tobacco composed of, or derived from, tobacco lamina, or a mixture of tobacco lamina and tobacco stem). For example, a representative blend may incorporate 30 to 70 parts burley tobacco (e.g., lamina, or lamina and stem), and 30 to 70 parts flue cured tobacco (e.g., stem, lamina, or lamina and stem) on a dry weight basis. Other example tobacco blends incorporate 75 parts flue-cured tobacco, 15 parts burley tobacco, and 10 parts Oriental tobacco; or 65 parts flue-cured tobacco, 25 parts burley tobacco, and 10 parts Oriental tobacco; or 65 parts flue-cured tobacco, 10 parts burley tobacco, and 25 parts Oriental tobacco; on a dry weight basis. Other example tobacco blends incorporate 20 to 30 parts Oriental tobacco and 70 to 80 parts flue-cured tobacco on a dry weight basis.

Tobacco materials used in the present disclosure can be subjected to, for example, fermentation, bleaching, and the like. If desired, the tobacco materials can be, for example, irradiated, pasteurized, or otherwise subjected to controlled heat treatment. Such treatment processes are detailed, for example, in US Pat. No. 8,061,362 to Mua et al., which is incorporated herein by reference. In some embodiments, tobacco materials can be treated with water and an additive capable of inhibiting reaction of asparagine to form acrylamide upon heating of the tobacco material (e.g., an additive selected from the group consisting of lysine, glycine, histidine, alanine, methionine, cysteine, glutamic acid, aspartic acid, proline, phenylalanine, valine, arginine, compositions incorporating di- and trivalent cations, asparaginase, certain non-reducing saccharides, certain reducing agents, phenolic compounds, certain compounds having at least one free thiol group or functionality, oxidizing agents, oxidation catalysts, natural plant extracts (e.g., rosemary extract), and combinations thereof. See, for example, the types of treatment processes described in US Pat. Pub. Nos. 8,434,496, 8,944,072, and 8,991,403 to Chen et al., which are all incorporated herein by reference. In some embodiments, this type of treatment is useful where the original tobacco material is subjected to heat in the processes previously described.

In some embodiments, the type of tobacco material is selected such that it is initially visually lighter in color than other tobacco materials to some degree (e.g., whitened or bleached). Tobacco pulp can be whitened in some embodiments according to any means known in the art. For example, bleached tobacco material produced by various whitening methods using various bleaching or oxidizing agents and oxidation catalysts can be used. Example oxidizing agents include peroxides (e.g., hydrogen peroxide), chlorite salts, chlorate salts, perchlorate salts, hypochlorite salts, ozone, ammonia, potassium permanganate, and combinations thereof. Example oxidation catalysts are titanium dioxide, manganese dioxide, and combinations thereof. Processes for treating tobacco with bleaching agents are discussed, for example, in US Patent Nos. 787,611 to Daniels, Jr.; 1,086,306 to Oelenheinz; 1,437,095 to Delling; 1,757,477 to Rosenhoch; 2,122,421 to Hawkinson; 2,148,147 to Baier; 2,170,107 to Baier; 2,274,649 to Baier; 2,770,239 to Prats et al.; 3,612,065 to Rosen; 3,851,653 to Rosen; 3,889,689 to Rosen; 3,943,940 to Minami; 3,943,945 to Rosen; 4,143,666 to Rainer; 4,194,514 to Campbell; 4,366,823, 4,366,824, and 4,388,933 to Rainer et al.; 4,641,667 to Schmekel et al.; 5,713,376 to Berger; 9,339,058 to Byrd Jr. et al.; 9,420,825 to Beeson et al.; and 9,950,858 to Byrd Jr. et al.; as well as in US Pat. App. Pub. Nos. 2012/0067361 to Bjorkholm et al.; 2016/0073686 to Crooks; 2017/0020183 to Bjorkholm; and 2017/0112183 to Bjorkholm, and in PCT Publ. Appl. Nos. WO1996/031255 to Giolvas and WO2018/083114 to Bjorkholm, all of which are incorporated herein by reference.

In some embodiments, the whitened tobacco material can have an ISO brightness of at least 50%, at least 60%, at least 65%, at least 70%, at least 75%, or at least 80%. In some embodiments, the whitened tobacco material can have an ISO brightness in the range of 50% to 90%, 55% to 75%, or 60% to 70%. ISO brightness can be measured according to ISO 3688:1999 or ISO 2470-1:2016.

In some embodiments, the whitened tobacco material can be characterized as lightened in color (e.g., "whitened") in comparison to an untreated tobacco material. White colors are often defined with reference to the International Commission on Illumination's (CIE's) chromaticity diagram. The whitened tobacco material can, in some embodiments, be characterized as closer on the chromaticity diagram to pure white than an untreated tobacco material.

Typical inclusion ranges for tobacco materials can vary depending on the nature and type of the tobacco material, and the intended effect on the final mixture, with an example range of up to 30% by weight (or up to 20% by weight or up to 10% by weight or up to 5% by weight), based on total weight of the mixture (e.g., 0.1 to 15% by weight). In some embodiments, a tobacco material (e.g., a whitened tobacco material) is included in a relatively small amount (e.g., 0.01% to 0.1% by weight). In some embodiments, the products of the disclosure can be characterized as completely free or substantially free of tobacco material (other than purified nicotine as an active ingredient), as referenced herein below.

### Other additives

Other additives can be included in the disclosed mixture. For example, the mixture can be processed, blended, formulated, combined and/or mixed with other materials or ingredients. The additives can be artificial, or can be obtained or derived from herbal or biological sources. Examples of further types of additives include thickening or gelling agents (e.g., fish gelatin), emulsifiers, oral care additives (e.g., thyme oil, eucalyptus oil, and zinc), preservatives (e.g., potassium sorbate and the like), zinc or magnesium salts selected to be relatively water soluble for compositions with greater water solubility (e.g., magnesium or zinc gluconate) or selected to be relatively water insoluble for compositions with reduced water solubility (e.g., magnesium or zinc oxide), disintegration aids, or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No. 2007/0062549 to Holton, Jr. et al., each of which is incorporated herein by reference. Typical inclusion ranges for such additional additives can vary depending on the nature and function of the additive and the intended effect on the final mixture, with an example range of up to 10% by weight, based on total weight of the composition (e.g., 0.1% to 5% by weight).

The aforementioned additives can be employed together (e.g., as additive formulations) or separately (e.g., individual additive components can be added at different stages involved in the preparation of the final mixture). Furthermore, the aforementioned types of additives may be encapsulated as provided in the final product or mixture. Example encapsulated additives are described, for example, in WO2010/132444 to Atchley, which has been previously incorporated by reference herein.

In some embodiments, the disclosed compositions can comprise (in addition to the nicotine component(s) described), a filler component (e.g., MCC), a base (e.g., NaOH), a sweetener (e.g., xylitol, sucralose, and/or acesulfame K, or the like), a salt, and a flavorant. In some embodiments, the composition comprises: 0% to 1.5% of freebase nicotine; 2% to 8% by weight of a nicotine polymer complex (e.g., comprising 20% nicotine); 3% to 6% by weight of a base; 2% to 8% by weight of a salt, 30% to 50% by weight of a filler, 1% to 5% by weight of a sweetener, and 0.5% to 2.5% by weight of a flavorant, all based on the total weight of the composition within the pouched product, including the additional water sprayed onto the pouched product after pouching, as described further herein below.

Generally, the products of the disclosure can have widely varying nicotine release rates. The pH of the composition may, in some embodiments, affect the rate of release of nicotine from the nicotine-polymer complex. Dissolution and counterions confirms that pH impacts the extraction of nicotine from the resin. For example, reducing the pH of the composition may slow the release of nicotine therefrom. However, as referenced herein above, the inclusion of certain alkali metal or alkaline earth metal salts can enhance release of nicotine from the resin, even at low pH values.

As noted above, in some embodiments, the inclusion of two nicotine components in some embodiments can provide for two different rates of release of nicotine from a given product within the oral cavity. For example, the first nicotine component (which may provide for somewhat immediate and/or fast release) may be released more quickly than the second nicotine component (which may provide for delayed and/or extended release of nicotine). By "immediate" release in some embodiments is meant that release begins substantially immediately upon introduction of the product into the oral cavity (or upon contact with saliva) or shortly thereafter. By "delayed" release in some embodiments is meant that the nicotine may not begin substantially immediately upon introduction of the product into the oral cavity. By "fast" release in some embodiments is meant that the release of a substantial portion of nicotine occurs in a relatively short period of time following introduction of the product into the oral cavity. By "extended" release in some embodiments is meant that release occurs over a more prolonged period of time following introduction of the product into the oral cavity.

In some embodiments, the nicotine is released from the disclosed compositions/products within the user's oral cavity over a period of at least 30 minutes, at least 40 minutes, at least 45 minutes, at least 50 minutes, at least 55 minutes, or at least 60 minutes, e.g., 30 minutes to 120 minutes, 30 minutes to 90 minutes, 30 minutes to 60 minutes, 40 minutes to 120 minutes, 40 minutes to 90 minutes, 40 minutes to 60 minutes, 45 minutes to 120 minutes, 45 minutes to 90 minutes, 45 minutes to 60 minutes, 50 minutes to 120 minutes, 50 minutes to 120 minutes, 50 minutes to 90 minutes, 50 minutes to 80 minutes, or 50 minutes to 70 minutes. In some embodiments, 80% or more of the nicotine is released from the pouched product after 90 minutes of use. In some embodiments, a nicotine polymer complex as described herein can be described as exhibiting extended release of the nicotine therefrom. In some embodiments, this extended release exhibited in some embodiments by a nicotine polymer complex as provided herein is extended relative to nicotine release from a comparable nicotine polymer complex, e.g., prepared by conventional means. In some embodiments, this extended release exhibited in some embodiments by a nicotine polymer complex as provided herein is extended relative to the release of one or more other nicotine components (where present) from a product.

The water content of the products provided herein can vary. In some embodiments, the water content of the disclosed pouched products is above 5% by weight, e.g., 5% to 80%, 70%, 60%, or 50%. In some embodiments, the water content of the disclosed pouched products is 48% or below (e.g., including 5% to 48%). In some embodiments, the products have a water content that is above 15% by weight, above 20% by weight, above 25% by weight, above 30% by weight, or above 40% by weight, based on the entirety of the pouched product. Certain examples of total water content according to some embodiments include 5% to 80%, 5% to 50%, 5% to 48%, 10% to 50%, 10% to 48%, 15% to 50%, 15% to 48%, 25% to 35%, 25% to 50%, 25% to 48%, 30% to 50%, 30% to 48%, 30% to 35%, 40% to 50%, 40% to 48%, 45% to 50%, or 45% to 48% by weight. Such total water content includes, e.g., water in the composition within the pouched composition and additional water added to the product, *e*.*g*., sprayed onto the outside of the product after pouching. The moisture content (e.g., including water plus humectant) can also vary and can, in some embodiments, fall within the ranges noted above for water content.

In some embodiments, any one or more of a filler component, a tobacco material, and the overall oral product described herein can be described as a particulate material. As used herein, the term "particulate" refers to a material in the form of a plurality of individual particles, some of which can be in the form of an agglomerate of multiple particles, wherein the particles have an average length to width ratio less than 2:1, such as less than 1.5:1, such as 1:1. In some embodiments, the particles of a particulate material can be described as substantially spherical or granular.

### Preparation of the compositions/products

The manner by which the various components of the mixture are combined may vary. As such, the overall mixture of various components with e.g., powdered mixture components may be relatively uniform in nature. The components noted above, which may be in liquid or dry solid form, can be admixed in a pretreatment step prior to mixture with any remaining components of the mixture, or simply mixed together with all other liquid or dry ingredients.

In some embodiments, the nicotine component/first nicotine component (i.e., the nicotine polymer complex) may not be water soluble; as such, in some embodiments, the order of mixing may be relevant. In some embodiments, a composition is provided as follows. Dry ingredients, including the filler and the first nicotine component (i.e., the nicotine-polymer complex) are combined to give a dry phase. Wet ingredients, including the second nicotine component (e.g., in aqueous solution form) and flavorant are separately combined to give a liquid phase. Additional ingredients, such as sweeteners are added to the liquid phase. The dry phase and liquid phase are mixed; a base is added to the mixture, e.g., while blending. Additional water is generally added to the pouches during pouching, as referenced herein below, to achieve the desired moisture content.

The various components of the mixture to be pouched may be contacted, combined, or mixed together using any mixing technique or equipment known in the art. Any mixing method that brings the mixture ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference. In some embodiments, the components forming the mixture are prepared such that the mixture thereof may be used in a starch molding process for forming the mixture. Manners and methods for formulating mixtures will be apparent to those skilled in the art. See, for example, the types of methodologies set forth in US Pat. No. 4,148,325 to Solomon et al.; US Pat. No. 6,510,855 to Korte et al.; and US Pat. No. 6,834,654 to Williams, US Pat. Nos. 4,725,440 to Ridgway et al., and 6,077,524 to Bolder et al., each of which is incorporated herein by reference.

In some embodiments, a moisture-permeable packet or pouch can act as a container for use of the composition within. For example, the pouch provides a liquid-permeable container of a type that may be considered to be similar in character to the mesh-like type of material that is used for the construction of a tea bag. If desired, flavoring ingredients, disintegration aids, and other desired components, may be incorporated within, or applied to, the pouch material. The composition/construction of such packets or pouches, such as the container pouch 20 in the embodiment illustrated in FIG. 4, may be varied as noted herein.

The pouches can be formed from a fleece material, e.g., fibrous nonwoven webs. As used herein, the term "fiber" is defined as a basic element of textiles. Fibers are often in the form of a rope- or string-like element. As used herein, the term "fiber" is intended to include fibers, filaments, continuous filaments, staple fibers, and the like. The term "multicomponent fibers" refers to fibers that comprise two or more components that are different by physical or chemical nature, including bicomponent fibers. Specifically, the term "multicomponent fibers" includes staple and continuous fibers prepared from two or more polymers present in discrete structured domains in the fiber, as opposed to blends where the domains tend to be dispersed, random or unstructured.

A "fleece material" as used herein may be formed from various types of fibers (e.g., cellulosic fibers; such as viscose fibers, regenerated cellulose fibers, cellulose fibers, and wood pulps; cotton fibers; other natural fibers; or polymer/synthetic-type fibers) capable of being formed into a traditional fleece fabrics or other traditional pouch materials. For example, fleece materials may be provided in the form of a woven or nonwoven fabric. Suitable types of fleece materials, for example, are described in U.S. Patent No. 8,931,493 to Sebastian et al.; US Patent App. Pub. No. 2016/0000140 to Sebastian et al.; and US Patent App. Pub. No. 2016/0073689 to Sebastian et al.; which are all incorporated herein by reference.

The term "nonwoven" is used herein in reference to fibrous materials, webs, mats, batts, or sheets in which fibers are aligned in an undefined or random orientation. The nonwoven fibers are initially presented as unbound fibers or filaments. One step in the manufacturing of nonwovens involves binding the various fibers or filaments together. The manner in which the fibers or filaments are bound can vary, and include thermal, mechanical and chemical techniques that are selected in part based on the desired characteristics of the final product, as discussed in more detail below.

In some embodiments, the pouch material can be dissolvable (i.e., orally ingestible) such that under conditions of normal use (i.e., upon contact with saliva in the mouth of a user), the pouch material dissolves. In some embodiments, the pouch material will dissolve after a significant amount of the soluble components of the composition within the pouch (e.g., active ingredient(s) and/or flavorant(s)) permeate through the pouch material into the mouth of the user. For example, the pouch material can be configured to dissolve at a rate such that the pouch material holds the composition together for a period of time sufficient to allow for the release of substantially all water soluble components. As described herein, in some embodiments, the composition within the pouch material can also be dissolvable. In some embodiments, the pouch material can be configured to dissolve at a rate similar to the rate at which the composition dissolves. In some embodiments, the pouch material can be adapted to or configured to at least partially dissolve or completely dissolve in 5 minutes or longer, 15 minutes or longer, 30 minutes or longer, or an hour or longer. In some embodiments, the pouch material can be adapted to or configured to at least partially dissolve or completely dissolve in no less than 30 minutes, no less than 45 minutes, or no less than an hour. In some embodiments, the pouch material may be adapted to or configured to at least partially dissolve or completely dissolve in a time of 30 seconds to 30 minutes, 1 minute to 25 minutes, 5 minutes to 20 minutes, or 5 minutes to 15 minutes. Without being limited by theory, a pouched product comprising a dissolvable pouch material can provide environmental advantages.

In some embodiments, dissolvable pouch materials can include, but are not limited to, spun or nonwoven alginate fibers, gluten fibers, mini-perforated flat sheets derived from alginate, carrageenan, and other polymer binders, and combinations thereof. Without being limited by theory, the dissolution rate of the pouch material can be controlled by the use of cross-linking technology between alginate or pectin and calcium salts, for example. In some embodiments, the dissolvable pouch material can include fast dissolving fibers formed using an electrospinning process (e.g., solution-based electrospinning) with hydrophilic polymers. *See, e.g.,* the techniques and fibers disclosed in Asawahame, Chawalinee et al., Formation of Orally Fast Dissolving Fibers Containing Propolis by Electrospinning Technique, Chiang Mai J. Sci. 2015; 42(2), p. 469-480, which is herein incorporated by reference in its entirety.

In some embodiments, the fibers within the fleece material may include, but are not limited to, a polymer selected from the group consisting of polyglycolic acid, polylactic acid, polyhydroxyalkanoates, polycaprolactone, polybutylene succinate, polybutylene succinate adipate, and copolymers thereof. In some embodiments, the fibers within the fleece material may be selected from the groups consisting of wool, cotton, fibers made of cellulosic material, such as regenerated cellulose, cellulose acetate, cellulose triacetate, cellulose nitrate, ethyl cellulose, cellulose acetate propionate, cellulose acetate butyrate, hydroxypropyl cellulose, methyl hydroxypropyl cellulose, protein fibers, and the like. See also, the fiber types set forth in US Pat. Appl. Pub. No. 2014/0083438 to Sebastian et al., which is incorporated by reference herein. In some embodiments, the pouch material can include a polymer selected from the group consisting of polyvinylpyrrolidone, polyvinyl alcohol, and combinations thereof.

Regenerated cellulose fibers (e.g., viscose or lyocell fibers) can be particularly advantageous, and are typically prepared by extracting non-cellulosic compounds from wood, contacting the extracted wood with caustic soda, followed by carbon disulfide and then by sodium hydroxide, giving a viscous solution. The solution is subsequently forced through spinneret heads to create viscous threads of regenerated fibers. Example methods for the preparation of regenerated cellulose are provided in U.S. Pat. No. 4,237,274 to Leoni et al; U.S. Pat. No. 4,268,666 to Baldini et al; U.S. Pat. No. 4,252,766 to Baldini et al.; U.S. Pat. No. 4,388,256 to Ishida et al.; U.S. Pat. No. 4,535,028 to Yokogi et al.; U.S. Pat. No. 5,441,689 to Laity; U.S. Pat. No. 5,997,790 to Vos et al.; and U.S. Pat. No. 8,177,938 to Sumnicht, which are incorporated herein by reference. The manner in which the regenerated cellulose is made is not limiting, and can include, for example, both the rayon and the TENCEL processes. Various suppliers of regenerated cellulose are known, including Lenzing (Austria), Cordenka (Germany), Aditya Birla (India), and Daicel (Japan).

The fibers used in the nonwoven web according to the present disclosure can vary, and include fibers having any type of cross-section, including, but not limited to, circular, rectangular, square, oval, triangular, and multilobal. In some embodiments, the fibers can have one or more void spaces, wherein the void spaces can have, for example, circular, rectangular, square, oval, triangular, or multilobal cross-sections. As noted previously, the fibers can be selected from single-component (i.e., uniform in composition throughout the fiber) or multicomponent fiber types including, but not limited to, fibers having a sheath/core structure and fibers having an islands-in-the-sea structure, as well as fibers having a side-by-side, segmented pie, segmented cross, segmented ribbon, or tipped multilobal cross-sections.

The physical parameters of the fibers present in the nonwoven web can vary. For example, the fibers used in the nonwoven web can have varying size (e.g., length, dpf) and crimp characteristics. In some embodiments, fibers used in the nonwoven web can be nano fibers, submicron fibers, and/or micron-sized fibers. In some embodiments, fibers of the nonwoven webs useful herein can measure 1.5 dpf to 2.0 dpf, or 1.6 dpf to 1.90 dpf. In an example embodiment, each fiber can be a staple fiber. Each fiber length can measure 35 mm to 60 mm, or 38 mm to 55 mm, for example. In some embodiments, each fiber can measure 4-10 crimps per cm, or 5-8 crimps per cm. It can be advantageous for all fibers in the nonwoven web to have similar fiber size and crimp attributes to ensure favorable blending and orientation of the fibers in the nonwoven web.

The fibrous webs can have varying thicknesses, porosities and other parameters. The nonwoven web can be formed such that the fiber orientation and porosity of the pouched product formed therefrom can retain the composition adapted for oral use that is enclosed within the outer water-permeable pouch, but can also allow the flavors of the composition to be enjoyed by the consumer. For example, in some embodiments, the fibrous webs can have a basis weight of 20 gsm to 60 gsm, 20 gsm to 35 gsm, or 25 gsm to 30 gsm. In an example embodiment, the fibrous web can have a basis weight of 28 gsm. Basis weight of a fabric can be measured using ASTM D3776/D3776M-09a(2013) (Standard Test Methods for Mass Per Unit Area (Weight) of Fabric), for example. In various embodiments, the fibrous web can have a thickness of 0.1 mm to 0.15 mm (e.g., 0.11 mm). The fibrous web can have an elongation of 70% to 80%, e.g., 78%. In some embodiments, the fibrous web can have a peak load of 4 lbs. to 8 lbs., e.g., 5.5 lbs. Elongation and breaking strength of textile fabrics can be measured using ASTM D5034-09(2013) (Standard Test Method for Breaking Strength and Elongation of Textile Fabrics (Grab Test)), for example. In various embodiments, the fibrous web can have a Tensile Energy Absorption (TEA) of 35 to 40, e.g., 37. In some embodiments, the fibrous web can have a porosity of greater than 10,000 ml/min/cm². TEA can be measured, for example, as the work done to break the specimen under tensile loading per lateral area of the specimen. Porosity, or air permeability of textile fabrics can be measured using ASTM D737-04(2012) (Standard Test method for Air Permeability of Textile Fabrics), for example.

In some embodiments of the pouched product described herein, the outer water-permeable pouch is made from a nonwoven web as described above. In some embodiments, a pouch is constructed of a single layer of the nonwoven web. In some embodiments, the pouch material comprises a multilayer composite made up of two or more nonwoven layers, each layer being orally ingestible. Each nonwoven layer can be formed by processes discussed below. In a multilayer structure, a first layer can be relatively hydrophilic and a second layer can be relatively hydrophobic (compared to each other). In some embodiments, an outer water-permeable pouch can comprise an outer hydrophilic layer and an inner hydrophobic layer that can be in contact with the composition adapted for oral use. As such, the hydrophobic layer can, during storage of the pouched product, retain any moisture in the composition adapted for oral use such that flavors in the composition are not lost due to moisture loss. However, capillaries in the hydrophobic layer can wick out moisture into the mouth of the user, such that flavors are released into the oral cavity when used. In this manner, the pouch material can enhance storage stability without significantly compromising the enjoyment of the product by the end user. In some embodiments, the relatively hydrophilic layer could be located on the interior of the multi-layer structure. The two layers can be formed into a multi-layer composite nonwoven material using any means known in the art, such as by attaching the two layers together using adhesive or stitching. The hydrophobicity of a textile material can be evaluated, for example, by measuring the contact angles between a drop of liquid and the surface of a textile material, as is known in the art.

In some embodiments, the pouch material can comprise a flavor component (such as any of the flavor components noted herein), which can be applied to the nonwoven layer in any conventional manner such as by coating, printing, and the like. In some embodiments of a pouched product described herein, the flavor within an outer pouch material can differ from a flavor contained within the internal composition adapted for oral use. For example, in some embodiments, the pouch material can have a first flavor component and after the pouch material has dissolved, more moisture can reach the composition within the pouch material and a flavor component within the composition can be enhanced. In this manner, the product can be designed to provide multiple, different sensory experiences, a first sensory experience where the flavor in the outer pouch material transitions into the mouth of the user and a second sensory experience, typically occurring later in time, where the flavor of the internal composition transitions into the mouth of the user.

In some embodiments, a heat sealable binder coating or a binder material (e.g., a coating or other additive) may be added to the fibers prior to, during, or after forming the fleece material. As used herein, "heat sealable binder coatings" refers to coating materials, such as acrylic polymer compositions, applied to a substrate (e.g., a nonwoven web or fleece material) and which are capable of sealing seams of individual pouches upon heating. In some embodiments, a binder material can be added to the web fibers before or during the laying of the fibrous web (i.e., before the fibrous web is bonded to form a fleece material). In some embodiments, a binder material can be added to the fleece material after it has been formed. In some embodiments, the binder material is in the form of a liquid coating. In some embodiments, a binding powder can be applied to the fleece material. For example, powdered polyethylene can be used as a binder material. The liquid or powder coating can be applied, for example, between layers of fibers when cross-laying, air laying, or as an after treatment. A short exposure in an oven is sufficient to melt and fuse the binder material.

The means of producing the nonwoven web can vary. Web formation can be accomplished by any means known in the art. Web formation will typically involve a carding step, which involves deposition of the fibers onto a surface followed by aligning/blending the fibers in a machine direction. Thereafter, the fibrous web is typically subjected to some type of bonding/entanglement including, but not limited to, thermal fusion or bonding, mechanical entanglement, chemical adhesive, or a combination thereof. In some embodiments, the fibrous web is bonded thermally using a calendar (which can provide flat or point bonding), steam jet bonding, or a thru-air oven. Additional bonding methods include ultrasonic bonding and crimping. In some embodiments, needle punching is utilized, wherein needles are used to provide physical entanglement between fibers. In some embodiments, the web is entangled using hydroentanglement, which is a process used to entangle and bond fibers using hydrodynamic forces. As noted above, a binder material can be applied to the fibers of the fibrous web before laying the fibrous web, during formation of the fibrous web, and/or after the fibrous web has been bonded to form a fleece material. After forming the fleece material, heat can be applied to the fleece material in order to activate/at least partially melt the binder material to further bond the fleece material and thereby further enhance the mechanical integrity of the fleece material.

Methods for forming a nonwoven web comprising natural and synthetic fibers may include drylaid, airlaid and wetlaid methods. In some embodiments, the nonwoven fabric can be formed using a spunlaid or spunmelt process, which includes both spunbond and meltblown processes, wherein such processes are understood to typically entail melting, extruding, collecting and bonding thermoplastic polymer materials to form a fibrous nonwoven web. The technique of meltblowing is known in the art and is discussed in various patents, for example, U.S. Pat. Nos. 3,849,241 to Butin, 3,987,185 to Buntin et al., 3,972,759 to Buntin, and 4,622,259 to McAmish et al., each of which is herein incorporated by reference in its entirety. General spunbonding processes are described, for example, in U.S. Patent Nos. 4,340,563 to Appel et al., 3,692,618 to Dorschner et al.*,* 3,802,817 to Matsuki et al.*,* 3,338,992 and 3,341,394 to Kinney, 3,502,763 to Hartmann, and 30 3,542,615 to Dobo et al., which are all incorporated herein by reference.

In some embodiments, the nonwoven web is made by providing a dry laid or a spun laid web of fibers, and then needle punching the web to bond the dry laid or spun laid web. The needle punched fleece material is produced when barbed needles are pushed through the fibrous web, forcing some fibers upwards or downwards through the web by the barbed needles. The fibers punched through the web remain at their new position once the needles are withdrawn. This needling action interlocks fibers and holds the structure together by inter fiber friction forces caused by compression of the web, thereby bonding the web. By displacing a sufficient number of fibers in the web, the web is converted into a nonwoven fabric.

In some embodiments, the nonwoven web is made by a fleece carding process with point bonding. The point bonding (e.g., using a calendar) should be limited to a relatively small portion of the surface area of the nonwoven web to maintain good porosity in the web for migration of water-soluble components through the web during oral use. In some embodiments, the point bonding is limited to less than 60% of the surface area of the nonwoven web (or resulting pouch), such as less than 50%, less than 30%, or less than 20% (e.g., 1% to 50%, 5% to 40%, or 10% to 30%). An advantage of point bonding is the ability to control the porosity, flexibility and fabric strength.

In some embodiments, the nonwoven web can be subjected to hydroentangling. The term "hydroentangled" or "spunlaced" as applied to a nonwoven fabric herein defines a web subjected to impingement by a curtain of high speed, fine water jets, typically emanating from a nozzle jet strip accommodated in a pressure vessel often referred to as a manifold or an injector. This hydroentangled fabric can be characterized by reoriented, twisted, turned and entangled fibers. For example, the fibers can be hydroentangled by exposing the nonwoven web to water pressure from one or more hydroentangling manifolds at a water pressure in the range of 10 bar to 1000 bar. As compared to point bonding, spunlace technology, in some embodiments, will have less impact on porosity of the web and, thus, may enhance flavor transfer through the nonwoven pouch material.

In some embodiments, the nonwoven web can be subjected to a second bonding method in order to reduce elongation of the web during processing. In some embodiments, nonwoven webs of the present disclosure can exhibit significant elongation during high speed processing on pouching equipment. Too much elongation of the nonwoven web can cause the web to shrink during processing, such that the final product is not sized appropriately. As such, it can be necessary to modify process equipment to fit a wider roll of fleece, for example, to compensate for any shrinkage in the final product due to elongation.

In order to avoid or at least reduce such an elongation problem, in some embodiments the nonwoven web can be point bonded after the first bonding (e.g., hydroentangling) is completed. A second bonding process can increase the tensile strength of the nonwoven web and reduce elongation characteristics. For example, a point bonding process can bond a nonwoven web by partially or completely melting the web (e.g., the heat sealable binder material) at discrete points. For example, in some embodiments, the nonwoven web can be subjected to ultrasonic bonding after initial bonding of the web. Any ultrasonic bonding system for nonwoven materials known in the art can be used to ultrasonically bond the nonwoven web. See, for example, the apparatuses and devices disclosed in U.S. Pat. Nos. 8,096,339 to Aust and 8,557,071 to Weiler, incorporated by reference herein. In some embodiments, the nonwoven web can be subjected to point bonding via embossed and/or engraved calendar rolls, which are typically heated. See, e.g., the point bonding methods incorporating the use of very high calendar pressures and embossing techniques discussed in U.S. Pat. Publ. No. 2008/0249492 to Schmidt, herein incorporated by reference in its entirety. The point bonding process is typically limited to less than 60% of the surface area of the nonwoven web as noted above.

In some embodiments, the processing techniques used to blend, entangle and bond the nonwoven web can also impart a desired texture to the fibrous nonwoven web material. For instance, point bonding or hydroentangling can impart a desired texture (e.g. a desired pattern) to the nonwoven web. This textured pattern can include product identifying information. In some embodiments, the product identifying information is selected from the group consisting of product brand, a company name, a corporate logo, a corporate brand, a marketing message, product strength, active ingredient, product manufacture date, product expiration date, product flavor, product release profile, weight, product code (e.g., batch code), other product differentiating markings, and combinations thereof.

Various manufacturing apparatuses and methods can be used to create a pouched product described herein. For example, US Publication No. 2012/0055493 to Novak, III et al., incorporated by reference in its entirety, relates to an apparatus and process for providing pouch material formed into a tube for use in the manufacture of smokeless tobacco products. The pouch material can include a binder material according to the present disclosure (e.g., a binder material comprising an aliphatic polyester). Similar apparatuses that incorporate equipment for supplying a continuous supply of a pouch material (e.g., a pouch processing unit adapted to supply a pouch material to a continuous tube forming unit for forming a continuous tubular member from the pouch material) can be used to create a pouched product described herein. Representative equipment for forming such a continuous tube of pouch material is disclosed, for example, in U.S. Patent Application Publication No. US 2010/0101588 to Boldrini et al., which is incorporated herein by reference in its entirety. The apparatus further includes equipment for supplying pouched material to the continuous tubular member such that, when the continuous tubular member is subdivided and sealed into discrete pouch portions, each pouch portion includes a charge of a composition adapted for oral use. Representative equipment for supplying the filler material is disclosed, for example, in U.S. Patent Application Publication No. US 2010/0018539 to Brinkley, which is incorporated herein by reference in its entirety. In some instances, the apparatus may include a subdividing unit for subdividing the continuous tubular member into individual pouch portions and, once subdivided into the individual pouch portions, may also include a sealing unit for sealing at least one of the ends of each pouch portion. In other instances, the continuous tubular member may be sealed into individual pouch portions with a sealing unit and then, once the individual pouch portions are sealed, the continuous tubular member may be subdivided into discrete individual pouch portions by a subdividing unit subdividing the continuous tubular member between the sealed ends of serially-disposed pouch portions. Still in other instances, sealing (closing) of the individual pouch portions of the continuous tubular member may occur concurrently with, or within seconds of, the subdivision thereof, using a closing and dividing unit.

An example apparatus for manufacturing an oral pouch product is illustrated in FIGS. 1-5 of U.S. Publication No. 2012/0055493 to Novak, III et al.; however, this apparatus is used in a generic and descriptive sense only and not for purposes of limitation. It should also be appreciated that the following manufacturing process and related equipment is not limited to the process order described below. In some embodiments of the present disclosure, an apparatus similar to that described in U.S. Publication No. 2012/0055493 can be configured to removably receive a first bobbin on an unwind spindle assembly, the first bobbin having a continuous length of a material, such as a pouch material, wound thereon. When the first bobbin is engaged with the apparatus, the pouch material can be routed from the first bobbin to a forming unit configured to form a continuous supply of the pouch material into a continuous tubular member defining a longitudinal axis.

As such, as the pouch material is unwound from the first bobbin, the pouch material can be directed around an arrangement of roller members, otherwise referred to herein as a dancer assembly. A forming unit can be configured to cooperate with the first bobbin and the dancer assembly to take up slack in the pouch material and to maintain a certain amount of longitudinal tension on the pouch material as the pouch material is unwound from the first bobbin and fed to the forming unit, for example, by a drive system. One of ordinary skill in the art will appreciate that, between the first bobbin and the forming unit, the pouch material can be supported, routed, and/or guided by a suitably aligned series of any number of, for example, idler rollers, guideposts, air bars, turning bars, guides, tracks, tunnels, or the like, for directing the pouch material along the desired path. Typical bobbins used by conventional automated pouch making apparatuses often contain a continuous strip of pouch material of which the length may vary. As such, the apparatus described herein can be configured so as to handle bobbins of that type and size.

The forming unit can include one or more roller members configured to direct the pouch material about a hollow shaft such that the continuous supply of the pouch material can be formed into a continuous tubular member. The forming unit can include a sealing device configured to seal, fix, or otherwise engage lateral edges of the pouch material to form a longitudinally-extending seam, thereby forming a longitudinally-extending continuous tubular member. In some embodiments, an insertion unit can be configured to introduce charges of the composition adapted for oral use into the continuous tubular member through the hollow shaft. The insertion unit may be directly or indirectly engaged with the hollow shaft.

A leading edge or end (also referred to as a laterally-extending seam) of the continuous tubular member can be closed/sealed such that a charge of composition adapted for oral use inserted by the insertion unit, is contained within the continuous tubular member proximate to the leading end. The leading end can be closed/sealed via a closing and dividing unit configured to close/seal a first portion of the continuous tubular member to form the closed leading end of a pouch member portion. The closing and dividing unit can also be configured to form a closed trailing edge or end of a previous pouch member portion. In this regard, the closing and dividing unit can also be configured to close a second portion of the continuous tubular member to form the closed trailing end of the pouch member portion. In this regard, the closing and dividing unit can close the ends, by heat-sealing, or other suitable sealing mechanism.

As illustrated in FIGS. 20-22 of U.S. Publication No. 2012/0055493 to Novak, III et al., the closing and dividing unit can be configured to divide the continuous tubular member, between the closed trailing end and the closed leading end of serially-disposed pouch member portions, along the longitudinal axis of the continuous tubular member, and into a plurality of discrete pouch member portions such that each discrete pouch member portion includes a portion of the oral composition from the insertion unit. In this regard, the closing and dividing unit can include a blade, heated wire, or other cutting arrangement for severing the continuous tubular member into discrete pouch member portions. For example, the closing and dividing unit can include first and second arm members configured to interact to close and divide the continuous tubular member.

In operation, a charge of the composition adapted for oral use (i.e., an amount suitable for an individual pouch member portion) can be supplied to the pouch member portion by an insertion unit after a leading end has been closed, but prior to the closing of a trailing end. In some embodiments, after receiving the charge of the oral composition, the discrete individual pouch member portion can be formed by closing the trailing end and severing the closed pouch member portion from the continuous tubular member such that an individual pouched product is formed.

The amount of material contained within each pouch may vary. In various embodiments, the weight of the mixture within each pouch is at least 50 mg, for example, from 50 mg to 2 grams, from 100 mg to 1.5 grams, or from 200 mg to 700 mg. In certain smaller embodiments, the dry weight of the material within each pouch is at least 50 mg to 150 mg. For some larger embodiments, the dry weight of the material within each pouch does not exceed 300 mg to 500 mg. In some embodiments, each pouch/container may have disposed therein a flavor agent member, as described in greater detail in US Pat. No. 7,861,728 to Holton, Jr. et al., which is incorporated herein by reference. For example, at least one flavored strip, piece or sheet of flavored water dispersible or water soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32; which are incorporated herein by reference. It is noted that fill volume in some embodiments is 75% to 100%.

In some embodiments, the nonwoven web can be sufficiently tacky so as to create issues with high-speed pouching equipment. Therefore, in some embodiments, a Teflon coating, or similar material, can be applied to one or more surfaces of the pouching equipment that touch the nonwoven web such as, for example, rollers, cutting instruments, and heat sealing devices in order to reduce and/or alleviate any problems associated with the pouch material sticking to the pouching equipment during processing.

The pouched products can further include product identifying information printed or dyed on the outer water-permeable pouch or imprinted (e.g., embossed, debossed, or otherwise pressed) on the outer water-permeable pouch, such as described in U.S. Pat. Appl. Pub. No. 2014/0255452 to Reddick et al., filed March 11, 2013, which is incorporated by reference herein. As noted above, flavorants can also be incorporated into the nonwoven web if desired, such as by coating or printing an edible flavorant ink onto the nonwoven web. *See, e.g.,* U.S. Pat. Appl. Pub. Nos. 2012/0085360 to Kawata et al. and 2012/0103353 to Sebastian et al., each of which is herein incorporated by reference.

The disclosed pouched products can be provided in a range of sizes. In some embodiments, a largest dimension (length, "L") is 16 to 40 mm or 20 to 40 mm, e.g., 16 mm, 17 mm, 18 mm, 19 mm, 20 mm, 21 mm, 22 mm, 23 mm, 24 mm, 25 mm, 26 mm, 27 mm, 28 mm, 29 mm, 30 mm, 31 mm, 32 mm, 33 mm, 34 mm, 35 mm, 36 mm, 37 mm, 38 mm, 39 mm, or 40 mm. In some embodiments, the largest perpendicular dimension to the length (width, "W") is 8 to 20 mm or 10 to 20 mm, e.g., 8 mm, 9 mm, 10 mm, 11 mm, 12 mm, 13 mm, 14 mm, 15 mm, 16 mm, 17 mm, 18 mm, 19 mm, or 20 mm. Certain non-limiting embodiments have rough largest dimensions of 38 mm (length) × 18 mm (width); 37.5 mm (length) × 12 mm (width); 38 mm (length) × 12 mm (length); 33 mm (length) × 18 mm (width); 33 mm (length) × 12 mm (length), 31 mm (length) × 12 mm (width), 30 mm (length) × 12 mm (width), 29 mm (length) × 14 mm (width), 28 mm (length) × 13 mm (width), 28 mm (length) × 12 mm (width), 27 mm (length) × 16 mm (width), 24 mm (length) × 12 mm (width) and 22 mm (length) × 13 mm (width). The third dimension (thickness, T, not shown in FIG. 6), understood to represent the 3-dimensional thickness of the products, can vary. In some embodiments, the thickness can vary, e.g., from 1 mm to 20 mm or 2 mm to 10 mm, although the disclosure is not limited thereto. Certain examples of thicknesses include, e.g., 2 mm, 3 mm, 4 mm, 5 mm, 6 mm, 7 mm, 8 mm, 9 mm, 10 mm, 11 mm, 12 mm, 13 mm, 14 mm, 15 mm, 16 mm, 17 mm, 18 mm, 19 mm, or 20 mm at the pouch's thickest point. In some embodiments, the total length, width, and thickness of the pouched product is 130 mm or less, 120 mm or less, 110 mm or less, 100 mm or less, 90 mm or less, 80 mm or less, 70 mm or less, 60 mm or less, 50 mm or less, or 40 mm or less, e.g., 30 mm to 130 mm, 30 mm to 100 mm, 50 to 100 mm, or 50 to 70 mm. Advantageously, in such embodiments, the thickness of such pouched products is 8 mm or less. Surface area of certain pouches (defined as length times width × 2) is 900 mm² or less, 800 mm² or less, 700 mm² or less, 600 mm² or less, 500 mm² or less, 400 mm² or less, 300 mm² or less, 250 mm² or less, 200 mm² or less, or 150 mm² or less (e.g., with a minimum of 100 mm² in some embodiments).

In some embodiments, the disclosed pouches have a length L of 35 to 60 mm and a width W of 8 to 18 mm. Certain, non-limiting examples of pouches provided herein are as follows: a pouch with L ≥ 35 mm and W ≥ 8 mm, a pouch with L ≥ 35 mm and W ≥ 10 mm, a pouch with L ≥ 35 mm and W ≥ 12 mm, a pouch with L ≥ 35 mm and W ≥ 14 mm, a pouch with L ≥ 35 mm and W ≥ 16 mm, a pouch with L ≥ 40 mm and W ≥ 8 mm, a pouch with L ≥ 40 mm and W ≥ 10 mm, a pouch with L ≥ 40 mm and W ≥ 12 mm, a pouch with L ≥ 40 mm and W ≥ 14 mm, a pouch with L ≥ 40 mm and W ≥ 16 mm, a pouch with L ≥ 50 mm and W ≥ 8 mm, a pouch with L ≥ 50 mm and W ≥ 10 mm, a pouch with L ≥ 50 mm and W ≥ 12 mm, a pouch with L ≥ 50 mm and W ≥ 14 mm, and a pouch with L ≥ 50 mm and W ≥ 16 mm. Certain advantageous ranges of length and width of large pouches are, in some embodiments, a length L of 35 mm to 60 mm, such as 40 mm to 60 mm, 50 mm to 60 mm, 35 mm to 50 mm, and 35 mm to 40 mm, and a width W of 8 mm to 16 mm, such as 8 mm to 14 mm, 8 mm to 12 mm, 8 mm to 10 mm, 9 mm to 16 mm, 9 mm to 14 mm, 9 mm to 12 mm, 9 mm to 10 mm, 10 mm to 16 mm, 10 mm to 14 mm, 10 mm to 12 mm, or 14 to 16. In various embodiments, the total measurements for the length, width, and thickness (i.e., adding all four sides of the pouch, plus the thickness) are within the following ranges. In some embodiments, the total length, width, and thickness of a large pouch as provided herein is 90 mm or greater, 100 mm or greater, 110 mm or greater, 120 mm or greater, 130 mm or greater, 140 mm or greater, or 150 mm or greater. Advantageously, in such embodiments, the thickness of such pouches is 2 mm or greater (e.g., between 2 and 8 mm). Surface area of certain pouches (defined as length times width × 2) is 300 mm² or greater, 400 mm² or greater, 500 mm² or greater, 600 mm² or greater, or 700 mm² or greater (e.g., with a maximum of 1000 mm²), although the disclosure is not limited thereto.

By providing at least a portion of the nicotine within a pouched product in the form of a nicotine-polymer complex as described herein, various benefits can be afforded. For example, in some embodiments, such pouched products exhibit more controlled release of nicotine within the oral cavity of a user than corresponding products comprising the same amount of conventional nicotine-polymer complex. For example, the nicotine can, in some embodiments, be released over a longer period of time within the oral cavity. In some embodiments, a high proportion of protonated nicotine associated with the nicotine-polymer complex as included in some embodiments relative to freebase nicotine associated with the nicotine-polymer complex can give early nicotine release (particularly under low pH and ionic strength conditions). Without being bound by theory, it is believed that freebase nicotine may more strongly interact with acidic groups on the cationic exchange resin, requiring high pH and/or ionic strength conditions to fully release from the resin; protonated nicotine (as defined above) may have a less strong interaction with the resin, promoting nicotine under lower pH and ionic strength conditions (relative to freebase nicotine). Advantageously, by employing nicotine in the form of a nicotine-polymer complex as provided herein, release characteristics can be modified/adjusted through pH and ionic strength. By adjusting these parameters, the release characteristics of a given pouched product composition can be modified as desired.

A pouched product as described herein can be packaged within any suitable inner packaging material and/or outer container. See also, for example, the various types of containers for smokeless types of products that are set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference.

Products of the present disclosure configured for oral use may be packaged and stored in any suitable packaging in much the same manner that conventional types of smokeless tobacco products are packaged and stored. For example, a plurality of packets or pouches may be contained in a cylindrical container. The storage period of the product after preparation may vary. As used herein, "storage period" refers to the period of time after the preparation of the disclosed product. In some embodiments, one or more of the characteristics of the products disclosed herein (e.g., retention of whiteness, lack of color change, retention of volatile flavor components) is exhibited over some or all of the storage period. In some embodiments, the storage period (*i.e*., the time period after preparation) is at least one day. In some embodiments, the storage period is from 1 day, 2, or 3 days, to 1 week, or from 1 week to 2 weeks, from 2 weeks to 1 month, from 1 month to 2 months, from 2 months to 3 months, from 3 months to 4 months, or from 4 months to 5 months. In some embodiments, the storage period is any number of days between 1 and 150. In some embodiments, the storage period may be longer than 5 months, for example, at least 6 months, at least 7 months, at least 8 months, at least 9 months, at least 10 months, at least 11 months, or at least 12 months.

### EXAMPLES

Various nicotine source materials and pouched products incorporating such nicotine source materials were prepared and evaluated according to the following methods.

### Preparation of nicotine-polymer complexes

**Material AA:** 162.17g of nicotine Polacrilex (20% nicotine) was mixed with 1028g of deionized water at an initial temperature of 141F. The mixture was stirred for 2 hours then 201g of 0.5M hydrochloric acid was added and the mixture was stirred for 12 hours. The resulting mixture was vacuum filtered and washed with 1000g deionized water. The material was dried at 40C for 48hr. The resulting material AA contained 3.81% moisture and 10% nicotine.

**Material BA:** 83.57g of material AA was mixed with 14.10g neat nicotine and 14.25g benzoic acid. The resulting material BA contained 16.40% moisture and 17.10% total nicotine content. The freebase nicotine to resin weight ratio was calculated to be 0.05.

**Material AB:** 164.2g of nicotine Polacrilex (20% nicotine) was mixed with 1006.8g of deionized water and 79.96g 2.5M NaOH at an initial temperature of 143F. The mixture was stirred for 1 hour then 409.8g of 0.5M hydrochloric acid was added and the resulting mixture was vacuum filtered and washed with 800g deionized water. The resulting material was dispersed in 1008.8g deionized water and 209.31g hydrochloric acid and stirred for 1 hour then vacuum filtered and washed with 800g deionized water. The material was dried at 40C for 48hr. The resulting material AB contained 5.09% moisture and 4.70% nicotine.

**Material BB:** 70.11g of material AB was mixed with 16.25g neat nicotine and 12.35g benzoic acid. The resulting material BB contained 16.06% moisture and 19.80% total nicotine content. The freebase nicotine to resin weight ratio was calculated to be 0.05.

### Nicotine release properties of materials BA and BB according to the present disclosure

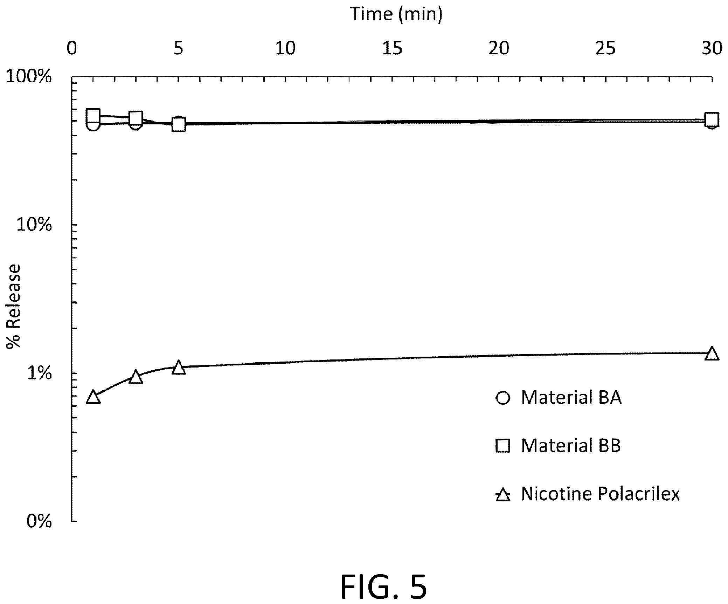

1.5g of materials BA, BB, and comparative material Nicotine Polacrilex (20% nicotine) were placed in individual beakers with 30g deionized water. Beakers were stirred at 400 rpm. 1mL of stirred solutions were sampled at 1, 3, 5, and 30 minutes. The samples were analyzed for nicotine content. FIG. 5 displays the percentage nicotine released from the materials. The percentage release is calculated from analyzed nicotine relative to the total amount of nicotine available on the material.

### Pouch preparation and evaluation

MO pouch fills CA (Table 2) and CB (Table 3) were made by mixing dry materials MCC, sodium chloride, and nicotine source (nicotine Polacrilex or Material BB) for 10 minutes in a Kitchen Aid mixer. All other ingredients were mixed into the water until dissolved. The water solution was then added to the dry ingredients and mixed for 10 minutes. The pH was measured by adding 1.5g of fill into 30mL water. pH was measured after 1h of stirring at 400rpm (Table 4).

**Table 2: Fill CA**

| Material | % |
|---|---|
| Microcrystalline cellulose | 43 |
| Sodium Chloride | 8 |
| Nicotine Polacrilex (20% N) | 19 |
| Water | 11 |
| Sodium Hydroxide | 13 |
| Xylitol | 3 |
| Ace-K | 1 |
| Ammonium Chloride | 1 |
| Flavor | 2 |

**Table 3: Fill CB**

| Material | % |
|---|---|
| Microcrystalline cellulose | 43 |
| Sodium Chloride | 8 |
| Material BB (25OCT22-1) | 23 |
| Water | 8 |
| Sodium Hydroxide | 13 |
| Xylitol | 3 |
| Ace-K | 1 |
| Ammonium Chloride | 1 |
| Flavor | 2 |

**Table 4: pH of MO fill materials CA, CB**

| Fill | pH |
|---|---|
| CA | 6.98 |
| CB | 6.61 |

Many modifications and other embodiments of the disclosure will come to mind to one skilled in the art to which this disclosure pertains having the benefit of the teachings presented in the foregoing description. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A method of preparing a nicotine-polymer complex, comprising contacting an exchange resin with a mixture of nicotine freebase and protonated nicotine.

2. The method of claim 1, wherein the exchange resin comprises a cationic exchange resin, or
wherein the exchange resin comprises polacrilex.

3. The method of claim 1, wherein the exchange resin is in the form of a nicotine-polymer complex, the method comprising:
washing the nicotine-polymer complex to remove at least a portion of nicotine present thereon and form a washed nicotine-polymer complex; and
contacting the washed nicotine-polymer complex with the mixture of nicotine freebase and protonated nicotine.

4. The method of claim 3, wherein the nicotine-polymer complex comprises nicotine associated with a cationic exchange resin, or
wherein the nicotine-polymer complex comprises nicotine polacrilex.

5. The method of claim 3, wherein the washing is done using water or wherein the washing is done using an acidic solution or an alkaline solution.

6. The method of any of claims 1 to 5, wherein the protonated nicotine is in the form of a nicotine ion-pair, or
wherein the protonated nicotine is nicotine benzoate.

7. The method of any of claims 1 to 5, wherein the protonated nicotine is in the form of a nicotine ion-pair complex, or
wherein the nicotine ion-pair complex is nicotine 3.2 molar excess benzoate.

8. The method of any of claims 1 to 5, wherein the protonated nicotine is in the form of a nicotine salt.

9. The method of any of claims 1 to 8, wherein the nicotine-polymer complex has a weight ratio of freebase nicotine to resin of 0 to 2, or
wherein the nicotine-polymer complex has a weight ratio of freebase nicotine to resin of 0 to 0.05.

10. The method of any of claims 1 to 9, further comprising preparing the mixture of nicotine freebase and protonated nicotine, or
further comprising preparing the mixture of nicotine freebase and protonated nicotine, wherein the preparing comprises mixing the nicotine freebase and the protonated nicotine, or
further comprising preparing the mixture of nicotine freebase and protonated nicotine, wherein the preparing comprises mixing the nicotine freebase and an agent capable of reacting with nicotine freebase to give the protonated nicotine.

11. The method of any of claims 1 to 10, wherein the mixture further comprises one or more processing aids selected from the group consisting of salts, humectants, buffers, and combinations thereof.

12. A nicotine-polymer complex, prepared by the method of any of claims 1 to 11.

13. A method of preparing a pouched product, comprising:
mixing a nicotine-polymer complex prepared according to the method of any of claims 1 to 11 with one or more fillers and optional additional components to provide a composition adapted for oral use; and
incorporating the composition within a cavity of an outer water-permeable pouch to form a pouched product.

14. The method of claim 13, the pouched product having a pH of 8 or greater; or having a pH of 6.5 to 8; or having a pH of 6.5 or below.

15. The method of claim 13 or 14, wherein the nicotine-polymer complex is a first nicotine component and the composition further comprises a second nicotine component selected from the group consisting of nicotine freebase, protonated nicotine, and a nicotine salt.
